# EUROPEAN PATENT SPECIFICATION

(11) **EP 1 877 141 B1**
(45) Date of publication and mention of the grant of the patent: **12.06.2013**
(21) Application number: 06724347.7
(22) Date of filing: 13.04.2006
(51) Int. Cl.: A61P 35/00, A61K 31/7028, A61K 31/22, A61K 31/255

(54) **SUBSTANCES AND PHARMACEUTICAL COMPOSITIONS FOR THE INHIBITION OF GLYOXALASES AND THEIR USE AGAINST BACTERIA**
SUBSTANZEN UND PHARMAZEUTISCHE ZUSAMMENSETZUNGEN FÜR DIE HEMMUNG VON GLYOXALASEN UND IHRE VERWENDUNG GEGEN BAKTERIEN
SUBSTANCES ET COMPOSITIONS PHARMACEUTIQUES POUR L'INHIBITION DE GLYOXALASES ET LEUR UTILISATION CONTRE DES BACTERIES

(30) Priority: 15.04.2005 DE 102005018641; 15.04.2005 DE 102005018642
(43) Date of publication of application: 16.01.2008
(73) Proprietor: Biomac Privatinstitut für medizinische und Zahnmedizinische Forschung, Entwicklung und Diagnostik GmbH, 04103 Leipzig (DE)
(72) Inventor: HUSE, Klaus, 04416 Markkleeberg (DE); BIRKENMEIER, Gerd, 04103 Leipzig (DE); BIRKENMEIER, Monika, 04103 Leipzig (DE)
(74) Representative: Kailuweit, Frank
(86) International application number: PCT/EP2006/003466
(87) International publication number: WO 2006/108681

(56) References cited:
- WO-A-01/68085
- WO-A-02/074301
- WO-A-03/088955
- WO-A-2005/004857
- DE-A1- 4 407 484
- US-A- 5 633 285
- US-A1- 2003 232 884
- US-A1- 2006 171 978
- MIYAJI TAKEHIKO ET AL: "Ethyl pyruvate decreases sepsis-induced acute renal failure and multiple organ damage in aged mice." KIDNEY INTERNATIONAL. NOV 2003, vol. 64, no. 5, November 2003 (2003-11), pages 1620-1631, XP002405486 ISSN: 0085-2538
- HALL S S ET AL: "Synthesis and evaluation of alpha-hydroxythiol esters as antitumor agents and glyoxalase I inhibitors." JOURNAL OF MEDICINAL CHEMISTRY. OCT 1977, vol. 20, no. 10, October 1977 (1977-10), pages 1239-1242, XP002405487 ISSN: 0022-2623
- PATENT ABSTRACTS OF JAPAN vol. 008, no. 056 (C-214), 14 March 1984 (1984-03-14) & JP 58 213716 A (JIYUNICHI IWAMURA), 12 December 1983 (1983-12-12)

## Description

### FIELD OF THE INVENTION

The invention relates to compounds of the general formula (I) for use in the treatment of bacterial infections in an animal. In one embodiment, the compounds of the general formula (I) are for the inhibition of glyoxalase I and/or II. The invention also relates to, pharmaceutical compositions comprising one or more compounds according to formula (I) and the use of one or more compounds according to formula (I) for the preparation of a medicament.

The compound, pharmaceutical compositionor medicament of the invention are for the treatment of bacterial infections.

The invention further relates to the prevention and/or combat of biofilms.

### BACKGROUND OF THE INVENTION

Bacteria are ubiquitous and essential for human and animal well-being, e.g. as part of the symbiotic gut flora, or for skin and mucosal homeostasis. Similarly, bacteria are known to colonise plants, and oftentimes are important symbionts of plants. Widely used classification schemes for bacteria relate to the makeup of their cell walls (Gram negative, Gram positive), or their oxygen requirements (aerobic, anaerobic, facultatively anaerobic).

A wide variety of bacteria, belonging to any of these categories, has the capacity to cause disease in higher organisms. For example, anaerobic germs are medically relevant and particularly dangerous (e.g. Clostridia). Disease causing bacteria can either be such that do not normally colonise this host, or can be part of the normal flora, which, however, lost its homeostasis.

Unchecked bacterial colonisation is a leading cause of diseases in all higher organisms. Bacterial infections affect each individual, and oftentimes lead to severe, or even lethal disease. Bacteria can directly destroy host tissue, and/or produce toxic metabolites, which contribute to the clinical manifestation of the infection.

Bacterial infections can be localized, e.g. the infection of a wound, or systemic. There is no organ or tissue which cannot be attacked by bacteria. Infections of the mucosa and the intestine are very common, as are wound infections. Bacterial infections can be acute, such as Streptococcal infection of the throat mucosa. Oftentimes infections can also be chronic. A classical example is the infestation of the gastrointestinal tract, in particular the gastric mucosa, by Helicobacter pylori, which causes chronic inflammation and discomfort. Chronic Helicobacter infection is a leading cause of gastric ulcers, and can even lead to gastric cancer. Helicobacter infections are difficult to treat by conventional antibiotics, and require administration of a cocktail of antibiotics over a long period of time.

Another chronic infection is Lyme disease, caused by systemic Borrelia infections, which can ultimately lead to various symptoms including joint disease and neurological symptoms. Further examples comprise tuberculosis, caused by Mycobacterium tuberculosis, which is also difficult to treat, requires long time administration of antibiotics and is known for increasing development of resistances.

Moreover, bacterial infection contributes to the development of chronic inflammation of periodontal tissue, which leads to atrophy of tissue surrounding teeth, and ultimately to the loss of teeth. Different anaerobic organisms are known to cause periodontosis. New insight into this disease is provided by the sequencing of the genome of Porphyromonas gingivalis in 2001, representing the first genome of a gram-negative anaerobic microorganism.

Porphyromonas is regarded as an important pathogen in the context of periodontosis, the inflammation of the periodontal ligament, associated with the high risk for subsequent loss of teeth. This bacterium develops resistances against many antibiotics and easily acquires resistance properties of other bacteria. In the USA alone, about 35 million people are effected by periodontosis.

Bacterial infections thus are a medical problem of foremost importance in humans, as well as animals and plants. Consequently, the discovery of antibiotics to control bacterial infections has been one of the most important medical breakthroughs achieved by mankind.

Bacteria as prokaryotes show metabolic and structural differences to their eukaryotic hosts, which can represent points of attack for a selective therapy. Such points are for example the synthesis of the cell wall and the specific translation system, which are targets for antibiotic interference. Antibiotics are substances produced by microorganisms, or produced synthetically, which inhibit or even kill other microorganisms. Additionally, they can be modified by chemical modifications to vary from the original substances. Such antibiotics are for example penicillins, aminocglycosides (streptomycins), tetracyclines, macrolides and many others. In a broader sense also synthetic substances like fluoroquinolones and sulfonamides belong to these antibiotics.

Increasingly, however, antibiotic control of bacterial infections becomes difficult, because bacteria rapidly acquire resistances to commonly used antibiotics. As a consequence, infectious diseases that could be successfully treated with antibiotics in the middle of the 20th century are today difficult to treat. For example, the recurrence of tuberculosis is a medical problem of renewed relevance in developing as well as industrialized countries (Konietzko, 1996).

Moreover, common antibiotic therapies, and in particular combination regimens, are in part known to result in severe side effects.

Bacteria have the ability to spread genetic material, such as elements that carry the mutations required for antibiotic resistance by mobile genetic elements (e.g. plasmids, transposons) in microbiological populations. This results in a permanent demand to adapt therapeutically interventions.

In this context it is detrimental that most of the antibiotics used in the clinic belong to a limited number of structural families of substances, e.g. the penicillins, cephalosporins, macrolide, etc. In these families of structurally related antibiotics, an original drug is varied in a limited number of positions to tailor the drug to specific needs.

The close structural relationship of many antibiotics has the effect that oftentimes bacteria develop resistances against a whole class of antibiotics. In addition, bacteria being resistant to multiple classes of antibiotics are known, in particular from the clinical and agricultural settings. Because of the ability of bacteria to exchange genetic material, resistance can spread rapidly.

The appearance of bacterial strains being "multi-drug-resistant" makes the use of known groups of substances even more complicate and an urgent need for new effective substances exists.

For example, bacteria, notably Staphylococcus aureus spp. become resistant to methicillin (MRSA) and other more common antibiotics such as oxacillin, penicillin and amoxicillin. MRSA has been emerging worldwide as one of the most important pathogens in hospital and healthcare facilities such as nursing homes and dialysis centers. There is only a limited number of drugs such as daptomycin available for treatment of MRSA (Cunha, 2005).

A further problem concerns resistance due to expression of beta-lactamases, mainly in E. coli, Klebsiella pneumoniae, Proteus mirabilis and Enterobacteriaceae spp. Beta-lactamases are enzymes produced by bacteria which hydrolyse important beta-lactam-antibiotics such as oxyimino-cephalosporins and aminoglycosides, fluoroquinolones and piperacillin-tazobactam (Turner, 2005). Moreover, beta-lactamases with extended specificity (ESBLs) have been isolated, which are less sensitive or even resistant to common antibiotics and beta-lactamase inhibitors (Westphal et al., 2000).

Many bacteria are capable of forming biofilms. Biofilms form when bacteria adhere to surfaces in an aqueous environment and begin to secrete slimy, glue-like substances into the extracellular space. These substances provide a matrix for the bacteria, and can anchor them to all kinds of materials e.g. metals, plastics, soil particles, medical material and living substrates such as teeth.

The formation of biofilms by bacteria moreover provides an ideal "breeding ground" for other microorganisms. Thus, biofilms oftentimes represent a complex microhabitat populated by a diverse range of microorganisms. Usually, biofilms harbour many species of bacteria, and sometimes in addition fungi, algea, protozoa, debris and corrosion products. Biofilm-forming bacteria colonise tube systems like pumps and filter systems (biofouling) for water distribution, water tanks, clinical equipment like catheters, wound dressing material etc. Communities of microbial cells develop on substrates of all kinds as well as on the surface of almost all aquatic ecosystems; they contain e.g. bacteria and fungi (Lens and Oflaherty, 2003).

Biofilms are particularly difficult to attack with conventional antibiotics. Antimicrobial efficacy against biofilms is limited as bacteria in biofilms are less susceptible to antimicrobial challenges. Biofilms can require 100 to 1000 times the concentration of an antibiotic to control infection.

Thus, infections associated with biofilms (e.g. inhaled biofilm fragments derived from contaminated inhalation devices, etc) are a particularly difficult to handle clinical problem. Moreover, contamination of substrates and systems by bacteria stemming from biofilms poses a significant technical and financial problem.

Thus, biofilms not only are of clinical importance in the context of bacterial infections and contamination of medical instruments and equipment, but bear further industrial significance, e.g. in colonisation of ship hulls, cooling water systems, oil recovery systems and corrosion of pipes (biocorrosion).

Therefore, new substance classes for use against bacteria, including drug resistant bacteria and biofilms, are urgently needed.

### SUMMARY OF THE INVENTION

The problem underlying the invention thus resides in providing new substances, compositions, and medicaments for use as anti-bacterial agents and methods of treatment using the same.

Accordingly, the present invention provides compounds of the general formula (I) 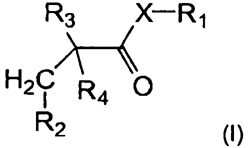 wherein X is O or S; and
R1 is a branched or non-branched alkyl, cycloalkyl, branched or non-branched alkenyl, cycloalkenyl, branched or non-branched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =O,
or R3 is OH and R4 is H; or R3 is H and R4 is OH
for use in the treatment of bacterial infections
with the proviso that when X is O, R2 is H and R3 or R4 is OH, R1 is not ethyl.

The anti-bacterial effects encompass bactericidal and bacteristatic action. Examples of bacteria against which the substances of the invention can be used comprise aerobic and/or anaerobic bacteria, Gram positive and/or gram negative bacteria, and bacteria forming biofilms.

According to the invention, the bacterial infection is one or more selected from an airway infection, comprising upper and lower airway infections, skin infection, intestinal infection, gastric infection, systemic infection, comprising tissue and blood infections, or is parodontosis.

The invention comprises also the use of the substances of the invention for the non-therapeutical prevention and or combating of biofilms, advantageously biofilms formed on a support in an aquatic environment. Said support can comprise one or more selected from metals, plastics, soil particles, medical material, medical devices or tissues, including living tissues such as teeth. In one embodiment the biofilm is associated with dental plaque.

Thus, the invention encompasses methods of preventing, combating or preventing and combating biofilms, comprising the use of a substance of the invention.

One embodiment relates to substances according to formula (I), wherein R1 comprises 1 to 8 carbon atoms and R2 is H or comprises 1 to 8 carbon atoms, or wherein R1 comprises 1 to 4 carbon atoms and R2 is H or comprises 1 to 2 carbon atoms.

In a further embodiment, the substance according to formula (I) is a substance wherein R1 and/or R2 is methyl, ethyl, propyl, isopropyl, butyl, or isobutyl. Particular embodiments according to the invention comprise one or more selected from methyl pyruvate, ethyl pyruvate, isopropyl pyruvate, butyl pyruvate, isobutyl pyruvate, ethyl 2- oxobutyrate, or the said pyruvate compound according to formula (I) wherein X = S, as well as compounds exemplified in table 1.

The invention also relates to substances according to formula (I), wherein in said substance R3 or R4 is OH and it is selected from the group comprising the D-, L-enantiomer, and the racemic mixture thereof (equimolar as well as non-equimolar), as exemplified in table 2.

The invention further relates to pharmaceutical compositions comprising one or more substances according to formula (I) and the use of said substances for the manufacture of a medicament.

In one embodiment, the pharmaceutical composition or medicament comprises one or more additional pharmaceutically active ingredients. Such ingredients can be selected from antibacterial agents, in particular antibiotics such as one or more selected from ß-lactam antibiotics, azithromycin, aminoglycosides, tetracyclines, macrolide, quinolones and fluoroquinolones, sulfonamides, oxazolidones, biocidal peptides, trimethoprim-sulfamethoxazole, cloramphenicol, vancomycin, metronidazol and rifampin.

The pharmaceutical composition or medicament can further comprise one or more auxiliary substances, including, but not limited to, fillers, flavouring agents and stabilizers. The pharmaceutical composition or medicament of the invention can be prepared in the form of galenic formulations commonly known in the art, including sustained release or controlled release galenic formulation.

The pharmaceutical composition or medicament of the invention is for topic or systemic administration, more particularly, for oral, intravenous, subcutaneous, intramuscular, intradermal, intraperitonal, rectal, intranasal, epidural, percutanous, transdermal, or pulmonary administration, or for administration as an aerosol, via mini-pumps, as mouth lavage, cream, ointment, spray, oil, gel, plaster, and/or via microbubbles. The pharmaceutical composition or medicament can also be in the form of a food supplement or a beverage supplement.

The pharmaceutical composition or medicament is for the treatment and/or prophylaxis of bacterial infections in animals, including invertebrates, non-mammalian vertebrates, mammalians and humans. The bacterial infection can be resistant to antibiotic treatment, such as beta-lactamases, antibiotica transporter molecules, RNA methylation, and/or is a resistance to methicillin and /or that resistance is due to genetic changes of the bacteria and may be an opportunistic infection.

The bacterial infection may be caused by one or more selected from the list comprising bacteria belonging to the genus Acrobacter, Actinobacillus, Actinomyces, Bacteroides, Borrelia, Bacillus, Brucella, Campylobacter, Clamydia, Clostridium, Corynebacterium, Cryptococcus, Enterobacter, Enterococcus, Erythrobacter, Eubacterium, Fusobacterium, gram-positiv cocci, Helicobacter, Hemophilus, Lactobacillus, Legionella, Listeria, Mycobacteria, Mycoplasma, Neissaria, Pasteurella, Peptostreptococcus, Pneumococcus, Porphyromonas, Prevotella, Pseudomonas, Rickettsia, Salmonella, Spirochetes (Borrelia, Treponema), Staphylococcus, Streptococcus, Vibrio, Yersinia, more specifically, Escherichia coli, Pneumocystis carinii, Helicobacter pylori or Borrelia burgdorferi.

In one embodiment, the bacteria are belonging to one or more of the genus Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococus aureus and Fusobacterium nucleatum.

In a further embodiment, the bacterium is Helicobacter, more specifically Helicobacter pylori.

In any of these embodiments, the infectious diseases may be an opportunistic infection, and/or may be characterized by antibiotic resistance.

In one embodiment, the infection is in an immunosuppressed animal, including man, wherein said immunosuppression is associated with hereditary or acquired immune-defects, comprising acquired immune defect associated with HIV, organ transplantation, chemotherapy or exposure to radiation.

In one embodiment, the animal, including man, is concomitantly suffering from a fungal or protozoal infection or worms, such as Trypanosoma, Leishmania, Plasmodium, Toxoplasma, helmithes, Candida spp., Aspergillus spp., Cryptococcus spp., Pneumocystis spp., Zygomyces spp., Dermatophytes, Blastomyces spp., Histoplasma spp., Coccidoides spp., Sporothrix spp., Microsporidia spp, Malassezia spp and Basidiomycetes. Some of these infectious diseases may be an opportunistic infection, and/or may be characterized by antibiotic resistance. In a further embodiment, the animal has a reduced blood glucose level.

According to the invention, the animal can be concomitantly suffering from cancer, and can be going to receive, is currently receiving, or has received conventional cancer therapy, comprising one or more of chemotherapy, surgery, radiotherapy or brachytherapy.

Moreover, the invention relates to contacting substrates with the substances of the invention as anti-bacterial agents, or incorporating the substances into compositions, wherein they act as anti-bacterial agents.

The mentioning of a particular embodiment in the context of one or more of a substance, pharmaceutical composition or medicament describes this embodiment for all these kinds of subject matter.

### DETAILED DESCRIPTION OF THE INVENTION

### A. Substances of the invention

In the context of this application, the terms "substances" or "compounds" are used interchangeably.

The present invention relates to compounds of the general formula (I), 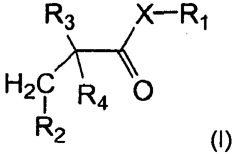 wherein X is O or S,
wherein R1 is a branched or non-branched alkyl, branched or non-branched alkenyl, branched or non-branched alkinyl, alkoxyalkyl, or alkoxycarbonylalkyl, each preferably with a chain length of C1 to C10, more preferably C1 to C8, more preferably C1 to C4, in particular C1, C2, C3 or C4;
or a cycloalkyl, cycloalkenyl, cycloalkinyl, aryl or a sugar residue, each preferably with a chain length of C3 to C10, more preferably C3 to C8, more preferably C3, C4, C5 or C6; and
R2 is H or a branched or non-branched alkyl, branched or non-branched alkenyl, branched or non-branched alkinyl, alkoxyalkyl, or alkoxycarbonylalkyl, each preferably with a chain length of C1 to C10, more preferably C1 to C8, more preferably C1 to C4, in particular C1, C2, C3 or C4;
or a cycloalkyl, cycloalkenyl, cycloalkinyl or aryl residue, each preferably with a chain length of C3 to C10, more preferably C3 to C8, more preferably C3, C4, C5 or C6; and R3 and R4 together are =O,
or R3 is OH and R4 is H; or R3 is H and R4 is OH.

In one embodiment, R1 is not ethyl when X is O, R2 is H and R3 or R4 is OH.

In one embodiment R1 is not methyl, ethyl, propyl or butyl, when the treated subject is a fish, X is O, and R3 and R4 together are =O.

In one embodiment the sugar in position R1 is substituted or non-substituted sugar.

In one embodiment R1 comprises 1 to 4 carbon atoms and R2 is H or comprises 1 or 2 carbon atoms. In a further embodiment of the substance according to formula (I), R1 and/or R2 is methyl, ethyl, propyl, isopropyl, butyl, or isobutyl.

In one embodiment R2 is H, and R1 is methyl, ethyl, propyl, isopropyl, butyl, or isobutyl.

Specific examples of compounds of the invention are listed in the following table 1, however, it is to be understood that this is not a limiting list. The skilled person can readily devise a large variety of additional compounds according to formula (I):

**Table 1: specific examples of compounds according to formula (I)**

| **R1** | **R2** | **R3 and R4** | **X** | **Name (I)** |
|---|---|---|---|---|
| **Alkyl** | | | | |
| Methyl | H | =0 | =O | Methyl pyruvate |
| Ethyl | H | =O | =O | Ethyl pyruvate |
| Ethyl | H | =O | =S | S-Ethyl 2-oxopropanethionate |
| Propyl | H | =O | =S | S-Propyl 2-oxopropanethionate |
| Butyl | H | =O | =S | S-Butyl 2-oxopropanethionate |
| Propyl | H | =O | =O | Propyl pyruvate |
| Butyl | H | =O | =O | Butyl pyruvate |
| Pentyl | H | =O | =O | Pentyl pyruvate |
| Hexyl | H | =O | =O | Hexyl pyruvate |
| Heptyl | H | =O | =0 | Heptyl pyruvate |
| Octyl | H | =O | =O | Octyl pyruvate |

| **Branched Alkyl** | | | | |
|---|---|---|---|---|
| Isopropyl | H | =O | =O | Isopropyl pyruvate |
| Isobutyl | H | =O | =O | Isobutyl pyruvate |
| Isopentyl | H | =O | =O | Isopentyl pyruvate |
| Isohexyl | H | =O | =O | Isohexyl pyrvate |
| Isoheptyl | H | =O | =O | lsoheptyl pyruvate |
| Isooctyl | H | =O | =O | Isooctyl pyruvate |

| **Cycloalkyl** | | | | |
|---|---|---|---|---|
| Cyclohexyl | H | =O | =O | Cyclohexyl pyruvate |
| Cyclohexyl methyl | H | =O | =O | Cyclohexylmethyl pyruvate |
| Cyclopentyl | H | =O | =O | Cyclopentyl pyrvate |
| Cyclopentyl methyl | H | =O | =O | Cyclopentylmethyl pyruvate |

| **Alkenyl residues** | | | | |
|---|---|---|---|---|
| Vinyl | H | =O | =O | Ethenyl pyruvate |
| Allyl | H | =O | =O | Propenyl pyruvate |
| Butenyl | H | =O | =O | Butenyl pyruvate |
| Pentenyl | H | =O | =O | Pentenyl pyruvate |
| Hexenyl | H | =O | =O | Hexenyl pyruvate |
| Heptenyl | H | =O | =O | Heptenyl pyruvate |
| Octenyl | H | =O | =O | Octenyl pyruvate |

| **Branched Alkenyl** | | | | |
|---|---|---|---|---|
| Isopropenyl | H | =O | =O | Isopropenyl pyruvate |
| Isobutenyl | H | =O | =O | Isobutenyl pyruvate |
| Isopentenyl | H | =O | =O | Isopentenyl pyruvate |
| Isohexenyl | H | =O | =O | Isohexenyl pyruvate |
| Isoheptenyl | H | =O | =O | lsoheptenyl pyruvate |
| IsooctenyL | H | =O | =O | Isooctenyl pyruvate |

| **Cycloalkenyl** | | | | |
|---|---|---|---|---|
| Cyclohexenyl | H | =O | =O | Cyclohexenyl pyruvate |
| Cyclohexenylmet hyl | H | =O | =O | Cyclohexenylmethyl pyruvate |
| Cyclo pentenyl | H | =O | =O | Cyclopentenyl pyruvate |
| Cyclopentenylme | H | =O | =O | Cyclopentenylmethyl pyruvate |
| thyl | | | | |

| **Alkinyl** | | | | |
|---|---|---|---|---|
| Ethinyl | H | =O | =O | Ethinyl pyruvate |
| Propinyl | H | =O | =O | Propinyl pyruvate |
| Butinyl | H | =O | =O | Butinyl pyruvate |
| Pentinyl | H | =O | =O | Pentinyl pyruvate |
| Hexinyl | H | =O | =O | Hexinyl pyruvate |
| Heptinyl | H | =O | =O | Heptinyl pyruvate |
| Octinyl | H | =O | =O | Octinyl pyruvate |

| **Branched Alkinyl** | | | | |
|---|---|---|---|---|
| Isopentinyl | H | =O | =O | Isopentinyl pyruvate |
| Isohexinyl | H | =O | =O | Isohexinyl pyruvate |
| Isoheptinyl | H | =O | =O | Isoheptinyl pyruvate |
| Isooctinyl | H | =O | =O | Isooctinyl pyruvate |

| **Cycloalkinyl** | | | | |
|---|---|---|---|---|
| Cyclooctinyl | H | =O | =O | Cyclooctinyl pyruvate |

| **Alkoxyalkyl** | | | | |
|---|---|---|---|---|
| Methoxymethyl | H | =O | =O | Methoxymethyl pyruvate |
| Ethoxymethyl | H | =O | =O | Ethoxymethyl pyruvate |
| Methoxyethyl | H | =O | =O | Methoxyethyl pyruvate |

| **Alkoxycarbonyl alkyl** | | | | |
|---|---|---|---|---|
| Methoxycarbonyl methyl | H | =O | =O | Methoxycarbonylmethyl pyruvate |
| Ethoxycarbonylm ethyl | H | =O | =O | Ethoxycarbonylmethyl pyruvate |

| **Aryl** | | | | |
|---|---|---|---|---|
| Phenyl | H | =O | =O | Phenyl pyruvate |
| Naphthyl | H | =O | =O | Naphthyl pyruvate |
| **Sugar** | H | | | |
| Glucosyl | H | =O | =O | Gucosyl pyruvate |
| Galactosyl | H | =O | =O | Galactosyl pyruvate |
| Mannosyl | H | =O | =O | Mannosyl pyruvate |

| **Alkyl** | **Alkyl** | | | |
|---|---|---|---|---|
| Methyl | Methyl | =O | =O | Methyl 2-oxobutanoat |
| Ethyl | Methyl | =O | =O | Ethyl 2-oxobutanoat |
| Ethyl | Ethyl | =O | =S | S-Ethyl 2-oxopentanethionate |
| Propyl | Methyl | =O | =O | Propyl 2-oxobutanoat |
| Butyl | Methyl | =O | =O | Butyl 2-oxobutanoat |
| Methyl | Ethyl | =O | =O | Methyl 2-oxopentanoate |
| Ethyl | Ethyl | =O | =O | Ethyl 2-oxopentanoate |
| Propyl | Ethyl | =O | =O | Propyl 2-oxopentanoate |
| Butyl | Ethyl | =O | =O | Butyl 2-oxopentanoate |
| Methyl | Propyl | =O | =O | Methyl 2-oxohexanoate |
| Ethyl | Propyl | =O | =O | Ethyl 2-oxohexanoate |
| Propyl | Propyl | =O | =O | Propyl 2-oxohexanoate |
| Butyl | Propyl | =O | =O | Butyl 2-oxohexanoate |
| Methyl | Butyl | =O | =O | Methyl 2-oxoheptanoate |
| Ethyl | Butyl | =O | =O | Ethyl 2-oxoheptanoate |
| Propyl | Butyl | =O | =O | Propyl 2-oxoheptanoate |
| Butyl | Butyl | =O | =O | Butyl 2-oxoheptanoate |

| **Branched Alkyl** | **Alkyl** | | | |
|---|---|---|---|---|
| Isobutyl | Methyl | =O | =O | Isobutyl 2-oxobutanoate |
| Isobutyl | Ethyl | =O | =O | Isobutyl 2-oxopentanoate |
| Isobutyl | Propyl | =O | =O | Isobutyl 2-oxohexanoate |
| Isobutyl | Butyl | =O | =O | Isobutyl 2-oxoheptanoate |

| **Cyclo Alkyl** | **Alkyl** | | | |
|---|---|---|---|---|
| Cyclohexyl | Ethyl | =O | =O | Cyclohexyl 2-oxopentanoate |
| Cyclohexyl methyl | Ethyl | =O | =O | Cyclohexylmethyl 2-oxopentanoate |

| **Sugar** | **Alkyl** | | | |
|---|---|---|---|---|
| Glucosyl | Methyl | =O | =O | Glucosyl 2-oxobutanoate |
| Glucosyl | Ethyl | =O | =O | Glucosyl 2-oxopentanoate |

| **Alkyl** | **Alkenyl** | | | |
|---|---|---|---|---|
| Propyl | Butenyl | =O | =O | Propyl 2-oxoheptenoate |

| **Cycloalkyl** | **Alkenyl** | | | |
|---|---|---|---|---|
| Cyclohexyl | Butenyl | =O | =O | Cyclohexyl 2-oxoheptenoate |

| **Alkyl** | **Alkinyl** | | | |
|---|---|---|---|---|
| Propyl | Butinyl | =O | =O | Propyl 2-oxoheptinoate |
| Butyl | Butinyl | =O | =O | Butyl 2-oxoheptinoate |

| **Cycloalkyl** | **Alkinyl** | | | |
|---|---|---|---|---|
| Cyclohexyl | Butinyl | =O | =O | Cyclohexyl 2-oxoheptinoate |

| **Alkoxyalkyl** | **Alkyl** | | | |
|---|---|---|---|---|
| Methoxymethyl | Ethyl | =O | =O | Methoxymethyl 2-oxopentanoate |
| Ethoxymethyl | Ethyl | =O | =O | Ethoxymethyl 2-oxopentanoate |
| Methoxyethyl | Ethyl | =O | =O | Methoxyethyl 2-oxopentanoate |

| **Alkoxycarbonyl alkyl** | **Alkyl** | | | |
|---|---|---|---|---|
| Methoxycarbonyl methyl | Ethyl | =O | =O | Methoxycarbonylmethyl 2-oxopentanoate |
| Ethoxycarbonylm ethyl | Ethyl | =O | =O | Ethoxycarbonylmethyl 2-oxopentanoate |

| **Alkyl** | **Alkoxycarbonyl alkyl** | | | |
|---|---|---|---|---|
| Ethyl | Methoxycarbonyl methyl | =O | =O | Ethyl-4-methoxycarbonyl-2-oxobutanoate |
| Ethyl | Ethoxycarbonylm ethyl | =O | =O | Ethyl-4-ethoxycarbonyl-2-oxobutanoate |

| **Alkyl** | **Alkoxyalkyl** | | | |
|---|---|---|---|---|
| Ethyl | Methoxy methyl | =O | =O | Ethyl 4-methoxy-2-oxobutanoate |
| Ethyl | Ethoxymethyl | =O | =O | Ethyl 4-ethoxy-2-oxobutanoate |
| Ethyl | Methoxyethyl | =O | =O | Ethyl 5-methoxy-2-oxopentanoate |

Particular examples of substances according to formula (I) comprise methyl pyruvate, ethyl pyruvate, propyl pyruvate, butyl pyruvate, pentyl pyruvate, hexyl pyruvate, octyl pyruvate, isobutyl pyruvate,isopentyl pyruvate, isohexyl pyruvate, isoheptyl pyrvate, isooctyl pyruvate, cyclopentyl pyruvate, cyclopentylmethyl pyruvate, cyclohexyl pyruvate, cyclohexylmethyl pyruvate, butenyl pyruvate, hexenyl pyruvate, isobutenyl pyruvate, isohexenyl pyruvate, butinyl pyruvate, hexinyl pyruvate, methoxymethyl pyruvate, ethoxymethyl pyruvate, ethoxycarbonylmethyl pyruvate, methyl-2-oxobutanoate, ethyl 2-oxobutanoate, butyl-2-oxo-butanoate, methyl-2-oxopentanoate, ethyl-2-oxoheptanoate, butyl-2-oxopentanoate, methyl-2-oxohexanoate, ethyl-2-oxohexanoate,butyl-2-oxohexanoate, methyl-2-oxoheptanoate, ethyl-2-oxoheptanoate, butyl-2-oxo-heptanoate, isobutyl-2-oxobutanoate, isobutyl-2-oxohexanoate, cyclohexyl-2-oxopentanoate, cyclohexylmethyl-2-oxopentanoate, propyl-2-oxoheptenoate, cyclohexyl-2-oxoheptenoate, butyl-2-oxoheptinoate, methoxymethyl-2-oxopantanoate, ethoxycarbonylmethyl-2-oxopentanoate,ethyl-4-methoxycarbonyl-2-oxobutanoate, ethyl-4-methoxy-2-oxobutanoate,
or the said compounds wherein X = S, and/or the said compound wherein R3 or R4 is OH.

Preferred examples of substances of the invention comprise methyl pyruvate, ethyl pyruvate, propyl pyruvate, butyl pyruvate, pentyl pyruvate, hexyl pyruvate,isopropyl pyruvate, isobutyl pyruvate, isopentyl pyruvate, isohexyl pyruvate, methyl-2-oxobutanoate, methyl-2-oxopentanoate, ethyl-2-oxobutanoate, butyl-2-oxobutanoate, ethyl-2-oxopentanoate.
cyclohexylmethyl pyruvate,
or the said compounds wherein X = S, and/or the said compound wherein R3 or R4 is OH.

More preferred compounds of the invention comprise methyl pyruvate, ethyl pyruvate, propyl pyruvate, butyl pyruvate, isobutyl pyruvate, ethyl-2-oxobutanoate, ethyl-2-oxopantanoate, cyclohexylmethyl pyruvate,
or the said compounds wherein X = S, and/or the said compound wherein R3 or R4 is OH.

Particularly preferred compounds are methyl pyruvate, ethyl pyruvate, butyl pyruvate, isobutyl pyruvate and ethyl-2-oxo-butyrate or the said compounds wherein X = S, in particular S-ethyl pyruvate, and/or the said compounds wherein R3 or R4 together are - OH.

### a) Substances according to formula (I) inhibit glyoxalases

### Surprisingly it was found that the substances according to formula (I) inhibit glyoxalase I and/or II.

Bacterial cells generate energy by the degradation of different food stuffs, and store it as chemical energy in energy rich compounds, particularly in the form of ATP. These energy rich compounds are subject to extensive turnover interconnected with anabolic and catabolic processes, by being used, for example, in the synthesis of proteins, nucleic acids, sugars, lipids etc., the transport of substances against concentration gradients and regulatory activities, and are formed anew in certain metabolic pathways. A plurality of compounds can serve as energy providing substances, the most important being sugars, amino acids, organic alcohols, alcohols and fatty acids. After metabolising the different monosaccharides and their di-, oligo- and polymers extra- or intracellularly into corresponding derivatives, sugar degradation takes place in glycolysis. Glycolysis allows anaerobic as well as, in combination with oxidative phosphorylation, aerobic energy generation.

Glycolysis, however, is always accompanied by the formation of oxoaldehydes, in particular of methylglyoxal. These compounds are highly toxic as they easily form adducts with cellular proteins and nucleic acids and lead to their inactivation. Therefore, all cells using glycolysis employ detoxification systems, in most cases consisting of the enzymes glyoxalase I and II.

Both glyoxalases I and II are responsible for the degradation of the side product of glycolysis, methylglyoxal. Methylglyoxal is cytotoxic (e.g. by the formation of adducts with cellular proteins and nucleic acids). Inhibition of the degradation of methylglyoxal leads to inhibition of cell proliferation and cell death by different mechanisms.

Accordingly, also in bacteria methylglyoxal is a widely occurring ketoaldehyde that is accumulated under physiological conditions and in particular under uncontrolled carbohydrate metabolism. Methylglyoxal synthesis can also be mediated by non-glycolytic enzymes, as are methylglyoxal synthase and amine oxidases, which are involved in acetone metabolism, and amino acid breakdown, respectively.

Thus, in one embodiment the substances according to formula (I) are for the inhibition of glyoxalase I and / or II, advantageously I and II. The inhibition of multiple enzymes drastically reduces the probability of developing resistance within the therapeutic period.

The compounds of the invention inhibit glyoxalase I and / or II, advantageously I and II. The inhibition of multiple enzymes drastically reduces the probability of developing resistance within the therapeutic period.

Surprisingly it was found that compounds of the present invention like e.g. ethyl pyruvate are capable of inhibiting glyoxalase I as well as glyoxalase II. Inhibition of glyoxalases by compounds of the present invention inhibits the cellular detoxification of methylglyoxal and via various mechanisms leads to the inhibition of cell proliferation and to cell death. Advantageously, compounds of the invention inhibit such cells showing a clearly increased rate of glycolysis whereas the metabolism of cells with a normal rate of glycolysis is not or only slightly affected.

Glyoxalase I (GLOI, alternatively abbreviated as Gly I,) is also known as (R)-S-lactoylglytythione methyl-glyoxal-lyase EC4.4.1.5), glyoxalase II (GLOII, alternatively abbreviated as Gly II) is also known as S-2-hydroxy-acylglutathione hydrolase (EC 3.1.2.6). A glyoxalase III has been described in E.coli. Its existence as a distinct enzyme is however to be shown.

Glyoxalases are phylogenetically highly conserved at the amino acid and genetic level. As used herein, the term "glyoxalase" refers to the mammalian enzymes glyoxalase I and/or II, as well as to the respective glyoxalases of non-mammalian eukaryotic and prokaryotic organisms, such as glyoxalase I and II of bacteria, fungi like yeast or other microorganisms.

Thus, the term "inhibiting glyoxalase I and/or II" encompasses the inhibition of the mammalian as well as the respective non-mammalian enzymes.

According to the invention, the substances according to formula (I) are for direct inhibition of glyoxalase I and/or II when R3 and R4 together are =O.

In contrast, when R3 or R4 are -OH, said substances according to formula (I) do not directly inhibit glyoxalase I and/or II. Rather, said substances, also called "prodrugs", are transformed, i.e. oxidized, to a substance wherein R3 and R4 together are =O.

Said transformation/oxidization can be effected ex vivo, e.g. by means of a chemical oxidant, such as potassium permanganate. Other suitable oxidants are for example hydrogen peroxide, iodine, iodide benzoic acid and others.

Alternatively, said transformation takes place in the organism, or on the skin or mucosa of the mammal upon administration of said compound. Such transformation is effected e.g. via dehydrogenases, in particular via lactate dehydrogenase (Lluis and Bozal, 1976).

Compounds of formula (I), wherein R3 or R4 is -OH, and which undergo transformation such that R3 and R4 together are =O are for example compounds of the general formula (II) and/or (III), 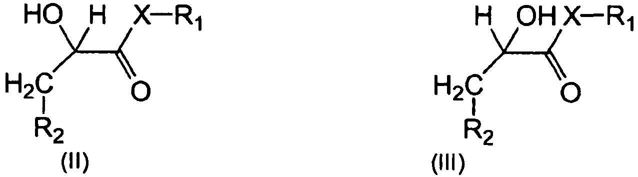 wherein X, R1 and R2 are defined as in formula (I), above.

Specific compounds of the general formula (II) and/or (III) are for example methyl lactate, propyl lactate, butyl lactate, ethyl lactate, and ethyl-2-hydroxybutanoate, which are transformed into, e.g. butyl pyruvate, ethyl pyruvate, and ethyl-2-oxobutanoate, respectively,

When in a substance according to formula (I) R3 or R4 is OH, the invention encompasses the D-, L- enantiomer and the racemic mixture thereof. In the context of this invention, equimolar as well as non-equimolar mixtures of corresponding enantiomers are to be considered as racemic mixtures.

In other words, in case the compounds of the invention are compounds with one or more chiral centres, for example ethyl lactate or butyl lactate, the corresponding D- and L-isomers can be used as well as racemic mixtures, for example ethyl D-lactate (DEL), ethyl L-lactate (LEL) or racemic mixtures of DEL and LEL, and butyl D-lactate (DBL), butyl L-lactate (LBL) or racemic mixtures of DBL and LBL, respectively.

Specific examples of compounds of the invention are listed in the following table 2, however, it is to be understood that this is not a limiting list. The skilled person can readily devise a large variety of additional compounds according to formula (II/III). The following table is understood to encompass the D- or L- Isomers or racemic mixtures of the listed substances. In other words, substances according to either formula II or formula III are specifically disclosed:

**Table 2: specific compounds according to formula II / III:**

| **R1** | **R2** | **R3/R4** | **X** | **Name** |
|---|---|---|---|---|
| **Alkyl** | | | | |
| Methyl | H | H/OH | =O | Methyl lactate |
| Ethyl | H | H/OH | =O | Ethyl lactate |
| Propyl | H | H/OH | =O | Propyl lactate |
| Butyl | H | H/OH | =O | Butyl lactate |
| Pentyl | H | H/OH | =O | Pentyl lactate |
| Hexyl | H | H/OH | =O | Hexyl lactate |
| Heptyl | H | H/OH | =O | Heptyl lactate |
| Octyl) | H | H/OH | =O | Octyl lactate |
| Ethyl | H | H/OH | =S | S-Ethyl 2-hydroxypropanethionate |
| Propyl | H | H/OH | =S | S-propyl 2-hydroxypropanethionate |
| Butyl | H | H/OH | =S | S-Butyl 2-hydroxypropanethionate |

| **Branched Alkyl** | | | | |
|---|---|---|---|---|
| Isopropyl | H | H/OH | =O | Isopropyl lactate |
| Isobutyl | H | H/OH | =O | Isobutyl lactate |
| Isopentyl | H | H/OH | =O | Isopentyl lactate |
| Isohexyl | H | H/OH | =O | Isohexyl lactate |
| Isoheptyl | H | H/OH | =O | Isoheptyl lactate |
| Isooctyl | H | H/OH | =O | Isooctyl lactate |

| **Cycloalkyl** | | | | |
|---|---|---|---|---|
| Cyclohexyl | H | H/OH | =O | Cyclohexyl lactate |
| Cyclohexylmethyl | H | H/OH | =O | Cyclohexylmethyl lactate |
| Cyclopentyl | H | H/OH | =O | Cyclopentyl lactate |
| Cyclopentyl methyl | H | H/OH | =O | Cyclopentylmethyl lactate |
| Cyclopropyl | H | H/OH | =O | Cyclopropyl lactate |
| Cyclopropyl methyl | H | H/OH | =O | Cyclopropylmethyl lactate |

| **Alkenyl** | | | | |
|---|---|---|---|---|
| Vinyl | H | H/OH | =O | Vinyl lactate |
| Allyl | H | H/OH | =O | Propenyl lactate |
| Butenyl | H | H/OH | =O | Butenyl lactate |
| Pentenyl | H | H/OH | =O | Pentenyl lactate |
| Hexenyl | H | H/OH | =O | Hexenyl lactate |
| Heptenyl | H | H/OH | =O | Heptenyl lactate |
| Octenyl | H | H/OH | =O | Octenyl lactate |

| **Branched Alkenyl** | | | | |
|---|---|---|---|---|
| Isopropenyl | H | H/OH | =O | Isopropenyl lactate |
| Isobutenyl | H | H/OH | =O | lsobutenyl lactate |
| Isopentenyl | H | H/OH | =O | Isopentenyl lactate |
| Isohexenyl | H | H/OH | =O | Isohexenyl lactate |
| Isoheptenyl | H | H/OH | =O | Isoheptenyl lactate |
| IsooctenyL | H | H/OH | =O | Isooctenyl lactate |

| **Cycloalkenyl** | | | | |
|---|---|---|---|---|
| Cyclohexenyl | H | H/OH | =O | Cyclohexenyl lactate |
| Cyclohexenylmethyl | H | H/OH | =O | Cyclohexenylmethyl lactate |
| Cyclopentenyl | H | H/OH | =O | Cyclopentenyl lactate |
| Cyclopentenylmethyl | H | H/OH | =O | Cyclopentenylmethyl lactate |

| **Alkinyl** | | | | |
|---|---|---|---|---|
| Ethinyl | H | H/OH | =O | Ethinyl lactate |
| Propinyl | H | H/OH | =O | Propinyl lactate |
| Butinyl | H | H/OH | =O | Butinyl lactate |
| Pentinyl | H | H/OH | =O | Pentinyl lactate |
| Hexinyl | H | H/OH | =O | Hexinyl lactate |
| Heptinyl | H | H/OH | =O | Heptinyl lactate |
| Octinyl | H | H/OH | =O | Octinyl lactate |

| **Branched Alkinyl** | | | | |
|---|---|---|---|---|
| Isopentinyl | H | H/OH | =O | Isopentinyl lactate |
| Isohexinyl | H | H/OH | =O | Isohexinyl lactate |
| Isoheptinyl | H | H/OH | =O | Isoheptinyl lactate |
| Isooctinyl | H | H/OH | =O | Isooctinyl lactate |

| **Cycloalkinyl** | | | | |
|---|---|---|---|---|
| Cyclooctinyl | H | H/OH | =O | Cyclooctinyl lactate |

| **Alkoxyalkyl** | | | | |
|---|---|---|---|---|
| Methoxymethyl | H | H/OH | =O | Methoxymethyl lactate |
| Ethoxymethyl | H | H/OH | =O | Ethoxymethyl lactate |
| Methoxyethyl | H | H/OH | =O | Methoxyethyl lactate |

| **Alkoxycarbonylalkyl** | | | | |
|---|---|---|---|---|
| Methoxycarbonylmeth yl | H | H/OH | =O | Methoxycarbonylmethyl lactate |
| Ethoxycarbonylmethyl | H | H/OH | =O | Ethoxycarbonylmethyl lactate |

| **Aryl** | | | | |
|---|---|---|---|---|
| Phenyl | H | H/OH | =O | Phenyl lactate |
| Naphthyl | H | H/OH | =O | Naphthyl lactate |

| **Sugar** | | | | |
|---|---|---|---|---|
| Glucosyl | H | H/OH | =O | Gucosyl lactate |
| Galactosyl | H | H/OH | =O | Galactosyl lactate |
| Mannosyl | H | H/OH | =O | Mannosyl lactate |

| **Alkyl** | **Alkyl** | | | |
|---|---|---|---|---|
| Methyl | Methyl | H/OH | =O | Methyl 2-hydroxybutanoate |
| Ethyl | Methyl | H/OH | =O | Ethyl 2-hydroxybutanoate |
| Propyl | Methyl | H/OH | =O | Propyl 2-hydroxybutanoate |
| Butyl | Methyl | H/OH | =O | Butyl 2-hydroxybutanoate |
| Methyl | Ethyl | H/OH | =O | Methyl 2-hydroxypentanoate |
| Ethyl | Ethyl | H/OH | =O | Ethyl 2-hydroxypentanoate |
| Ethyl | Ethyl | H/OH | =S | S-Ethyl 2-hydrxypentanethionate |
| Propyl | Ethyl | H/OH | =O | Propyl 2-hydroxypentanoate |
| Butyl | Ethyl | H/OH | =O | Butyl 2-hydroxypentanoate |
| Methyl | Propyl | H/OH | =O | Methyl 2-hydroxyhexanoate |
| Ethyl | Propyl | H/OH | =O | Ethyl 2-hydroxyhexanoate |
| Propyl | Propyl | H/OH | =O | Propyl 2-hydroxyhexanoate |
| Butyl | Propyl | H/OH | =O | Butyl 2-hydroxyhexanoate |
| Methyl | Butyl | H/OH | =O | Methyl 2-hydroxyheptanoate |
| Ethyl | Butyl | H/OH | =O | Ethyl 2-hydroxyheptanoate |
| Propyl | Butyl | H/OH | =O | Propyl 2-hydroxyheptanoate |
| Butyl | Butyl | H/OH | =O | Butyl 2-hydroxyheptanoate |

| **Branched Alkyl** | | | | |
|---|---|---|---|---|
| Isobutyl | Methyl | H/OH | =O | Isobutyl 2-hydroxybutanoate |
| Isobutyl | Ethyl | H/OH | =O | Isobutyl 2-hydroxypentanoate |
| Isobutyl | Propyl | H/OH | =O | Isobutyl 2-hydroxyhexanoate |
| Isobutyl | Butyl | H/OH | =O | Isobutyl 2-hydroxyheptanoate |

| **Cycloalkyl** | | | | |
|---|---|---|---|---|
| Cyclohexyl | Ethyl | H/OH | =O | Cyclohexyl 2-hydroxypentanoate |
| Cyclohexylmethyl | Ethyl | H/OH | =O | Cyclohexylmethyl 2-hydroxypentanoate |

| **Sugar** | **Alkyl** | | | |
|---|---|---|---|---|
| Glucosyl | Methyl | H/OH | =O | Glucosyl 2-hydroxybutanoate |
| Glucosyl | Ethyl | H/OH | =O | Glucosyl 2-hydroxypentanoate |

| **Alkyl** | **Alkenyl** | | | |
|---|---|---|---|---|
| Propyl | Butenyl | H/OH | =O | Propyl 2-hydroxyheptenoate |

| **Cycloalkyl** | **Alkenyl** | | | |
|---|---|---|---|---|
| Cyclohexyl | Butenyl | H/OH | =O | Cyclohexyl 2-hydroxaheptenoate |

| **Alkyl** | **Alkinyl** | | | |
|---|---|---|---|---|
| Propyl | Butinyl | H/OH | =O | Propyl 2-hydroxyheptinoate |
| Butyl | Butinyl | H/OH | =O | Butyl 2-hydroxyheptinoate |

| **Cycloalkyl** | **Alkinyl** | | | |
|---|---|---|---|---|
| Cyclohexyl | Butinyl | H/OH | =O | Cyclohexyl 2-hydroxyheptinoate |

| **Alkoxyalkyl** | **Alkyl** | | | |
|---|---|---|---|---|
| Methoxymethyl | Ethyl | H/OH | =O | Methoxymethyl 2-hydroxypentanoate |
| Ethoxymethyl | Ethyl | H/OH | =O | Ethoxymethyl 2-hydroxypentanoate |
| Methoxyethyl | Ethyl | H/OH | =O | Methoxyethyl 2-hydroxypentanoate |

| **Alkoxycarbonylalkyl** | **Alkyl** | | | |
|---|---|---|---|---|
| Methoxycarbonylmeth yl | Ethyl | H/OH | =O | Methoxycarbonylmethyl 2-hydroxypentanoate |
| Ethoxycarbonylmethyl | Ethyl | H/OH | =O | Ethoxycarbonylmethyl 2-hydroxypentanoate |

| **Alkyl** | **Alkoxycar bonylalkyl** | | | |
|---|---|---|---|---|
| Ethyl | Methoxycar bonylmethy I | H/OH | =O | Ethyl-4-methoxycarbonyl-2-hydroxybutanoate |
| Ethyl | Ethoxycarb onylmethyl | H/OH | =O | Ethyl-4-ethoxycarbonyl-2-hydroxybutanoate |

| **Alkyl** | **Alkoxyalky I** | | | |
|---|---|---|---|---|
| Ethyl | Methoxyme thyl | H/OH | =O | Ethyl 4-methoxy-2-hydroxybutanoate |
| Ethyl | Ethoxymethyl | H/OH | =O | Ethyl 4-ethoxy-2-hydroxybutanoate |
| Ethyl | Methoxyeth yl | H/OH | =O | Ethyl 5-methoxy 2-hydroxypentanoate |

The D- or L- enantiomers or the racemic mixtures thereof of the following substances are further particular examples of substances of the invention: methyl lactate, ethyl lactate, propyl lactate, butyl lactate, pentyl lactate, hexyl lactate, octyl lactate, isobutyl lactate, isopentyl lactate, isohexyl lactate, isoheptyl lactate, isooctyl lactate, cyclopentyl lactate, cyclopentylmethyl lactate, cyclohexyl lactate, cyclohexylmethyl lactate, butenyl lactate, hexenyl lactate, isobutenyl lactate,isohexenyl lactate, butinyl lactate, hexinyl lactate, methoxymethyl lactate, ethoxymethyl lactate, ethoxycarbonylmethyl lactate, methyl-2-hydroxybutanoate, ethyl 2-hydroxybutanoate, butyl-2-hydroxybutanoate, methyl-2-hydroxypentanoate, ethyl-2-hydroxyheptanoate, butyl-2-hydroxypentanoate, methyl-2-hydroxyhexanoate, ethyl-2-hydroxyhexanoate,butyl-2-hydroxyhexanoate, methyl-2-hydroxyheptanoate, ethyl-2-hydroxyheptanoate, butyl-2-hydroxyheptanoate,isobutyl-2-hydroxybutanoate, isobutyl-2-hydroxyhexanoate, cyclohexyl-2-hydroxypentanoate, cyclohexylmethyl-2-hydroxypentanoate, propyl-2-hydroxyheptenoate, cyclohexyl-2-hydroxyheptenoate, butyl-2-hydroxyheptinoate, methoxymethyl-2-hydroxypantanoate, ethoxycarbonylmethyl-2-hydroxypentanoate,ethyl-4-methoxycarbonyl-2-hydroxybutanoate, ethyl-4-methoxy-2-hydroxybutanoate,
or the said compounds, wherein X=S.

If ethyl lactate is used, ethyl L-lactate (LEL) as well as ethyl D-lactate (DEL) are effective.

The inventors' own measurements confirm the interconversion of ethyl lactate and ethyl pyruvate by NAD-dependent lactate dehydrogenases. Butyl lactate can, to a lesser degree than ethyl lactate, also be transformed by NAD-dependent lactate dehydrogenases. When butyl lactate is used according to the invention only cells with a particularly high activity of lactate dehydrogenase will reach therapeutically effective concentrations of butyl pyruvate.

Thus, compounds of the general formula (II) and (III) act as prodrugs, as exemplified in Examples 2 and 3.

Lactate and alkyl lactate, respectively, are transported over the cell membrane by a lactate shuttle (monocarboxylate transporters (MCT's)) (Garcia et al., 1994; von Grumbckow et al., 1999) in combination with a proton transporter. For the transport into mitochondria mitochondrial MCTs are available. Addition of lactate and its alkyl esters, respectively, to blood leads to slight alkalization due to the proton-connected lactate transporters whereas the application of pyruvate and its alkyl esters, respectively, leads to an acidosis of blood, caused by enzymatic ester cleavage. Lactate and alkyl lactate are transported stereo selectively and better through the membrane as compared to pyruvate and alkyl pyruvate (Roth and Brooks, 1990). Alkyl pyruvates administered to blood have to be transformed into alkyl lactates before they can enter cells.

Moreover, the rate of hydrolysis of compounds according to formula (I) wherein R3 or R4 is -OH is lower as compared to compounds wherein R3 and R4 together are =O, leading to an improved in vivo stability.

Therefore, it is advantageous to use compounds wherein R3 or R4 is -OH, and in particular, therapeutically active, physiologically compatible alkyl lactates.

In the context of this application, the compounds according to general formula (I), including direct glyoxalase inhibitors and their prodrugs, and the specific examples of compounds, including the compounds according to formula (II) and (III), are also summarily referred to as "compounds of the invention".

A particular advantage of the substances of the invention resides in the fact that toxicity of said substances and their metabolites is only very low (Clary et al., 1998). After saponification by esterases they are metabolized to equally non- or only slightly toxic alcohols and to carboxylic acids which are also produced in normal cell metabolism (e.g. pyruvate and lactate). For example, the concentration of lactate in human blood is 2-20 mM. Lactate is contained in many foods, is generated in metabolism and can be metabolised.

This also explains the low or even absent ecotoxicity of these compounds (Bowmer et al., 1998) in tests with Selenastrum capricornutum, Daphnia magna, Pimephales promelas and Brachydanio rerio. These compounds are also devoid of mutagenic potential in normal cells as demonstrated in an established test system (Andersen and Jensen, 1984).

### b) The substances of formula (I) differ from known inhibitors of glyoxalases

The inhibition of glyoxalases by compounds of the present invention has so far been unknown.

On the basis of the substrate of glyoxalase I, the hemithioacetal of methylglyoxal and glutathione, peptidic glyoxalase inhibitors are widely described in the literature (Creighton et al, 2003;; Hamilton & Creighton, 1992; Hamilton and Batist, 2004; Johansson et al., 2000; Kalsi et al., 2000; Kamiya et al, 2005; Ranganathan et al, 1995; Sharkey et al., 2000; Thornalley, 1993; Thornalley et al, 1996; Thornalley, 1996; Vince and Daluge, 1970).

US 4,898,870 describes pyrroloquinoline quinone compounds in the context of inhibition of glyoxalase I. WO 99/035128 also describes compounds for inhibition of glyoxalase I. WO 04/101506 describes a further class of non-peptidic inhibitors of glyoxalase I, as does Douglas et al, 1985.

However, the glyoxalase inhibitors known so far exhibit a relatively high or very high toxicity and are metabolized to compounds which in turn have manifold pharmacological effects, some of which lead to severe side effects.

Furthermore, the glyoxalase inhibitors known so far only inhibit either glyoxalase I or glyoxalase II, respectively. However, when inhibitors are directed to a single protein target only, resistance can develop very quickly, as for example mutations appear in the relevant protein, which make the inhibitor ineffective.

Therefore, the glyoxalase inhibitors of the present invention are advantageous over known inhibitors.

### c) The known effects of substances according to formula (I) do not encompass glyoxalase inhibition

From the known effect of methyl pyruvate its influence on glyoxalases was not predictable. For years methyl pyruvate has been intensely investigated as an insulinotropic compound (Düfer et al., 2002; Valverde et al., 2001; Lembert et al., 2001). This effect is mediated by influencing potassium channels and mitochondrial effects. Inhibitory effects on LDH have also been proposed (Lluis and Bozal, 1976).

Furthermore, it has been described that the administration of ethyl pyruvate can improve inflammatory states, reperfusion injury and ischemia (WO 03/088955; WO 02/074301; WO 01/024793, WO 05/044299). US 5,633,285A discloses a therapeutic cytoprotective-wound healing comprising pyruvate. WO2005/004857A1 discloses the use of pyruvate for the treatment of acute pancreatitis. In patent US2004/110833 ethyl pyruvate is used to influence cytokine mediated diseases. This is attributable to abolishing the effect of NF-kß (Han et al., 2005; Yang et al., 2004; Fink et al., 2004; Miyaji et al., 2003; Ulloa et al., 2002). However, opposite observations also exist in this respect (Mulier et al., 2005). WO01/68085A1 describes the use of isoleucine for stimulating the immune system and for prevention and treatment of infections. JP 58213716A describes a carcinostatic agent containing a compound with formula CH₃(CH₂)ₘ-A-COOR wherein A is -CH=CR'-, - CH₂CH(OH), or-CH(OH)CHR'-; R and R' are H or 1-10 C alkyl and m is 1-13. DE4407484 A1 describes a medicament for cancer therapy comprising substances which inhibit lactic acid secretion from cancer cells.

However, by no means these mechanisms indicate an inhibition of glyoxalases. Moreover, they can not be used to explain an inhibition of cell growth, because according to the findings of the present invention the growth of yeast cells and bacteria is also inhibited by ethyl pyruvate, which cells neither have NF-kß nor cytokines nor other inflammatory mediators.

Additionally, it could be shown that protein adducts of methylglyoxal, the concentration of which is increased after inhibition of glyoxalases, even increase the release of TNF-a and the activation of NF-kß (Fan et al., 2003). In particular, this mechanism can not be used to explain the inhibitory effect of ethyl pyruvate on proliferation as the effect of ethyl pyruvate on cytokines is also detectable when cells are not proliferating.

The inhibitory effect of ethyl pyruvate on proliferation mediated via the inhibition of glyoxalases is the more surprising as ethyl pyruvate, due to its known effect as "scavenger" of reactive oxygen radicals should rather have a growth enhancing effect (Varma et al., 1998). As a matter of fact, this has been described for normal human T-lymphocytes (Dong et al., 2005). In this report it has furthermore been described that the formation of the cytokine interleukin-2 was enhanced in these cells.

### B. Pharmaceutical composition/manufacture of a medicament

The present invention relates to the claimed medical use of compounds of the invention, their use for the preparation of medicaments and pharmaceutical compositions comprising said compounds.

In the following, particular embodiments will be described in the context of pharmaceutical compositions. However, it is to be understood that these embodiments also apply to the medical use of compounds of the invention and the manufacture of a medicament. In other words, any disclosure of an embodiment in the context of a pharmaceutical composition is not to be understood as being limited thereto, but also relates to the manufacture of a medicament. Thus, the terms "medical use of a compound of the invention", "pharmaceutical composition" and "use for the manufacture of a medicament" in the context of this application are interchangeable. This applies to the entirety of the present application.

The basic embodiment of the invention is a pharmaceutical composition comprising at least one substance of the invention.

In one embodiment, the pharmaceutical composition of the invention comprises the substance according to the invention as the sole active ingredient. Thus, in one embodiment the combination of the substance of the present invention with a further active ingredient is excluded. This does not exclude the presence of more than one substance of the present invention. This does also not exclude the presence of non-pharmaceutiaclly active additives, i.e. substances which contribute to preparing a galenic formulation, such as fillers, flavouring agents, stabilizers, etc.

In one embodiment the pharmaceutical composition can comprise a combination of one or more compounds of the general formula (I) wherein R3 and R4 together are =O, e.g. ethyl pyruvate, and one or more compounds wherein R3 or R4 is -OH like compounds of the general formula (II) and (III), e.g. ethyl lactate, (ethyl D- and/or L-lactate).

The pharmaceutical composition of the invention can further comprise one or more additional pharmaceutically active ingredients. In the context of combinations with further active ingredients the low toxicity of the compounds of the present invention as well as their metabolites is of particular advantage.

As further pharmaceutical compounds, preferably chemotherapeutics, immunosuppressive agents, common agents against worms and fungi, antibiotics, substances favoring cell differentiation like transcription- and growth factors, inhibitors of glycolysis or substrates for glycolysis are used.

For example, a combination of a compound of the present invention, such as ethyl pyruvate with common anti-bacterial agents, such as antibiotics comprising one or more selected from ß-lactam antibiotics (penicillins, ampicillins, carbenicillins, methicillin, ticarcillin, cephalosporin, imipenem, aztreonam), azithromycin, aminoglycosides (gentamycin, kanamycin, neomycin, netilmicin, streptomycin, tobramycin), tetracyclines (demeclocyclin, doxycyclin, minocyclin, oxytetracyclin), macrolide (azithromycin, clarithromycin, clindamycin, erythromycin, lincomycin), quinolones and fluoroquinolones (cinoxacin, nalidixic acid, ciprofloxacin, enoxacin, norfloxacin, levofloxacin, lomefloxacin), sulfonamides (sulfisoxazole, sulfamethoxazole, sulfadiazine, sulfacetamide), oxazolidones (linezolid), biocidal peptides (polymyxin, polymyxin B, colistin, bacitracin, like defensins), trimethoprim, trimethoprim-sulfamethoxazole, cloramphenicol, vancomycin, metronidazol rifampicin, rifampin, doxocyclin, gramicidin, cycloserin and isoniazid is preferably used.

A preferred combination consists of compounds of the present invention and an inhibitor of glycolysis wherein the inhibitor of glycolysis interferes with glycolysis downstream of the triosephosphate isomerase reaction. The rationale of such a combination is to increase the concentration of triosephosphates from which methylglyoxal evolves parametabolically or paracatalytically, and thus, to improve the efficacy of therapy.

Particularly preferred is the combination of compounds of the present invention, in particular ethyl pyruvate or the corresponding thioester, and oxamate, an inhibitor of lactate dehydrogenase. Also particularly preferred is the combination of compounds of the present invention and an inhibitor of the glycerol aldehyde phosphate dehydrogenase, such as iodide acetate, and/or the lactate dehydrogenase inhibitor oxamate.

Furthermore, chemotherapeutics in the context of a standard chemotherapy which generally exist for example for carcinomas and sarcomas can be used. Some representative examples of standard chemotherapeutic agents are cyclophosphamide and doxorubicin for the treatment of breast cancer and leukemia, taxol for the treatment of ovary cancer, and 5-fluorouracil or cisplatin for sarcoma.

In addition to the compounds according to the invention further compounds may be preferably applied which stimulate the metabolism of infectious organisms, such as bacteria, fungi or protozoa, like substrates of glycolysis, in particular glucose, or for example 2,4-dinitrophenol acting as uncoupler of the respiratory-chain. In this manner advantageously the concentration of methylglyoxal is increased further and the efficacy of the compounds of the invention is increased further, resulting in an enhanced efficacy of the pharmaceutical composition.

A further aspect of the invention is the use of compounds of the present invention in combination with known or novel genetic methods like siRNA and antisense nucleotides for the targeted inhibition of enzymes or proteins to increase the sensitivity of tumors (Nesterova and Cho-Chung, 2004).

The pharmaceutical composition or medicament can further comprise one or more auxiliary substances useful for the galenic formulations of drugs, including, but not limited to, fillers, flavouring agents, stabilizers and agents that prevent microbial growth in the pharmaceutical composition.

The pharmaceutical composition can be in any suitable galenic formulation, depending on the kind of disease to be treated and the chosen route of administration. The skilled person can readily select and prepare a suitable preparation on the basis of common general knowledge. Pharmaceutical compositions of the invention can be prepared according to known methods e.g. by mixing one or more effective substances with one or more carriers, and forming of e.g. tablets, capsules, or solutions. Where appropriate, solutions can be e.g. encapsulated in liposomes, microcapsules or other forms of containment.

Examples of suitable formulations comprise aqueous solutions which can optionally be buffered, water in oil emulsions, oil in water emulsions, creams, ointments and formulations comprising any of the foregoing.

The invention encompasses a pharmaceutical composition prepared in the form of a sustained release or controlled release galenic formulation. Such formulations allow the targeted release in e.g. a certain location, such as a certain part of the gut, or a certain tissue or organ, and/or allow the sustained release over a defined period of time.

A pharmaceutical preparation can also be prepared by mixing the ester components of the compounds of the invention under conditions at which compounds of the general formula (I) are formed. The pharmaceutical preparation can also be prepared by assembling ester components of the compounds of the invention such that in the organism, for example in the acidic environment of the stomach, the compounds of the general formula (I), (II) or (III) are formed. Ester components are for example an alkanol like for example ethanol and an organic acid like for example lactic acid.

The pharmaceutical composition of the invention comprises at least one compound of the invention in a therapeutically effective amount. The skilled person can readily determine the therapeutically effective amount in standard in vitro or in vivo experiments. For example, the effective amount can be estimated on the basis of an extrapolation from in vitro data, such as enzyme inhibition or cellular assays.

For example a dosage can be formulated in animal models which corresponds to the IC50 in cell culture experiments. Hence, according to commonly known methods, the optimal dosage for the vertebrate to be treated, such as humans, can be deduced from animal experiments. The amount of the agent to be administered naturally depends on the person to be treated, his body weight, his genetic and physical constitution, the disease state, the route of administration, the galenic formulation and other parameters.

Furthermore, dosage and the interval of administration can be guided by the individual plasma concentrations of the agent that guarantee a therapeutic effect.

Useful effective concentrations, i.e. concentrations to be achieved at the level of cellular exposure, range from at least 0.05 mM, preferably from 0.05 mM to 50 mM, more preferably 1 mM to 40 mM, more preferably 1 mM to 20 mM, most preferably 1 mM, 2.5 mM, 5 mM, or 7,5 mM in systemic application. In topic applications higher concentrations may be useful. Preferred are 0.2 to 200 mM, more preferred are 0.2 to 50 mM and 50 to 200 mM.

In other words, the concentrations above refer to desired blood and/or tissue concentrations, or local concentrations. Thus, the invention relates to pharmaceutical preparations suitable to achieve such concentrations upon administration.

To achieve a therapeutic effect the pharmaceutical composition of the present invention is generally applied for several days or weeks as repeated bolus doses (e.g. injections) or continuous administration (e.g. infusion), or any time period required to achieve a therapeutic effect, at the respective therapeutically effective dosage.

The pharmaceutical composition of the present invention can be administered topically or systemically. In both topical and systemic administration a local administration to a selected site can be performed. Because of their nature, esters are of limited stability, necessitating the use of higher and/or repeated doses for systemic application. This can be circumvented by local application.

Pharmaceutical compositions comprising the compounds of the invention can be administered according to generally known methods - including but not limited to oral, intravenous, intraarterial, subcutaneous, intramuscular, intradermal, intraperitonal, intraauricular, rectal, intranasal, epidural, percutanous, or transdermal administration, or administration as an aerosol, via mini-pumps, as mouth lavage, gel, ointment, cream, spray, oil, plaster, via microbubbles and/or pulmonary application (e.g. by inhalation).

Administration is for example systemic, e.g. by single or repeated oral or parenteral application, or via methods wherein the medicament is administered systemically in an inert vehicle and is only released at the desired location by respective manipulation. An example thereof is, amongst others, so called microbubbles as described in Bekeredjian et al. (2005) and Bekeredjian et al. (2003).

The pharmaceutical composition can also be a food supplement or beverage supplement. In the context of the present invention, "food supplement" or "beverage supplement" means a pharmaceutical composition that is administered together with the standard daily diet, or a special medical diet. It also means "health food", i.e. food of a particular composition that is consumed by subjects without medical supervision to achieve a prophylactic or therapeutic effect.

### C. Medical indications

The substance, pharmaceutical composition or medicament of the present invention is for use in the treatment of bacterial infections.

The invention encompasses the administration or use in invertebrates, non-mammalian vertebrates, mammals and humans in need thereof. In one embodiment the mammalian vertebrates are not dogs. In the context of this application, the term "animal" is meant to encompass non-vertebrate animals, vertebrate animals, comprising non-mammalian vertebrates and mammals, which mammals comprise man. Thus, the term animal encompasses humans.

According to the invention the cells proliferation of which is inhibited are bacteria. Such bacteria cause infectious diseases, such as various bacterial diseases.

### a) Bacterial infections

It was surprisingly found that the substances of the invention can be used for the treatment and/ or prophylaxis of bacterial infections, and more specifically, inhibition and/or killing of bacteria.

In one embodiment, the substance of the invention is not ethyl pyruvate when the disease is lethal polymicrobial bacterial peritonitis. In a further embodiment, the substance of the invention is not a substance according to formula (I) wherein R3 and R4 together are =O, when the disease is a cytokine mediated disease such as sepsis, septic shock or polymicrobial peritonitis.

The term "treatment and/or prophylaxis of bacterial infections" encompasses bactericidal and bacteristatic effects.

The effects on glyoxalases, and the associated anti-bacterial effects (i.e. bacteristatic and/or bactericidal effects) of substances of the present invention have previously been unknown.

US 5,580,902 discloses certain substances of the present invention as auxiliary agents in pharmaceutical compositions, in particular as enhancers for a multitude of active ingredients. This document does not, however, disclose an anti-bacterial effect of substances of the invention per se.

de Jaham (2003) discloses that ethyl lactate can be added to shampoos used in the treatment of canine superficial bacterial pyoderma. Prottey et al (1984), mentions ethyl lactate in the context of the treatment of acne. However, according to Prottey et al (1984), lactate, and not its ester, exhibits an effect on bacteria, by reducing skin pH. Therefore, no effect of ethyl lactate per se on bacteria is disclosed in this document. de Jaham (2003) does not provide any discussion of a putative mechanism of action, but cites the Prottey et al (1984), paper in this respect. Therefore, de Jaham (2003) does not contain disclosure beyond what is known from Prottey et al (1984).

A number of documents mentions substances of the invention in the context of cytokine mediated diseases, including sepsis or septic shock (e.g. Fink et al, 2004, Miyaji et al, 2003, Ulloa et al, 2002, Han et al, 2005, WO 03/088955, WO 02/074301, US2004/0110833). According to these documents, sepsis is a cytokine mediated clinical syndrome. For example, US2004/0110833 teaches that mediators released systemically by the innate immune system mediate the characteristic signs of sepsis, including microvascular hyperpermeability, coagulopathy, organ failure, tissue injury and lethal shock.

Therefore, these documents discuss an effect of substances of the invention on cytokine mediated pathologies. They do not, however, disclose or suggest any effects on bacteria per se. A direct effect on bacteria, and an effect on inflammatory cytokines represent two different clinical situations. The present invention is not intended to relate to the cytokine mediated sequelae of infection, such as sepsis or septic shock.

US 2005/0020678 discloses substances of the invention, as fungicides. It provides no teaching that could be generalized to other substances or applications, however.

Other known medical applications of substances of the invention equally provide no link to the treatment of bacterial infections.

WO 02/102366 describes certain pyruvate esters for the treatment of fish-parasites, such as plathelmintes. According to EP 0 717 984 and JP 8 208 422 proliferation of normal human cells, for example keratinocytes, is even stimulated by compounds of the present invention, which effect is used to improve the appearance of the skin. Similarly, in several patents (US 5,580,902; US 4,234,599; US 4,105,783) compounds of the present invention have been described as agents to improve skin consistency and smoothen wrinkles. Effects on keratosis, and several diseases characterized by defective keratinisation (dandruff, acne, palmar and plantar hyperkeratosis, dry skin, Darier's disease, lichen simplex chronicus, psoriasis, eczema, pruritus, warts and herpes) are described in US 3,879,537, US 3,920,835, US 4,246,261 and US 7,33,815.

Ethyl pyruvate has been used to improve cataracts (Devamanoharan et al., 1999). In this connection a lowering of dulcitol and glycated proteins by ethyl pyruvate has been found, connected to the effect of pyruvate formed by hydrolysis of ethyl pyruvate.

CN 1175632 describes ethyl lactate as an auxiliary substance in the manufacture of Spirulina wine, but does not disclose ethyl lactate as an active ingredient. WO 03/088955, WO 02/081020, US 2003/0232884 and WO 02/074301 deal with clinical situations such as reperfusion injury, kidney failure, ischemia and inflammatory disorders. Marx et al, (1988) suggests the inhibition of cancer cells by lactate. Stanko et al, (1994), discusses a role of pyruvate in the treatment of cancer. Hall et al. (1977) describe the synthesis and evaluation of alpha-hydroxythiol esters as antitumor agents.

Though not intending to be bound by theory, it is suggested that the anti bacterial activity of the compounds of the invention is due to their inhibition of glyoxalase I and/or II. Bacterial cells generally require significant amounts of energy for cell division and general metabolism, provided in the form of ATP. Glycolysis allows anaerobic as well as, in combination with oxidative phosphorylation, aerobic energy generation. In most bacteria mechanisms exist for preferring glucose metabolism (catabolite repression). Particularly when bacteria grow under anaerobic conditions, glycolysis is the most important way for energy generation.

Animals and humans regulate glucose concentration in their organs and body fluids in a narrow concentration range. The glucose concentration for example in human blood is constant at about 5 mM. Animals show a similar glucose homoeostasis.

Thus, most parasites, such as pathogenic bacteria, are specialized in the utilization of glucose in blood, body cavities and on the skin, and show a high rate of glycolysis. Degradation of glucose by glycolysis and the formation of oxoaldehydes are ubiquitous metabolic pathways, which are phylogenetically highly conserved (Heymans and Singh, 2003; Clugston et al., 1997).

Thus, glycolysis per se has been discussed as a possible therapeutic target (Brady and Cameron, 2004; Kavanagh et al., 2004; Lakhdar-Ghazal et al., 2002, Iwami et al., 1995).

However targeting glycolysis for therapeutic purposes has so far remained elusive, as glycolysis is a central metabolic pathway of parasites as well their hosts.

US 200410167079 describes for example a method for treatment of cancer by use of 2-deoxyglucose (2-DG), an inhibitor of glycolysis.

US 2003/0181393 discloses the glycolysis inhibitors 2-deoxyglucose, oxamat and iodide-acetate. Iodide acetate inhibits glycerolaldehyde-3-phosphate dehydrogenase and oxamate inhibits lactate dehydrogenase.

The main shortcoming of inhibiting glycolysis is that glycolysis is used for energy generation in almost all cells, such that healthy cells are also affected by inhibition of glycolysis. In particular the influence on the brain is dramatic as the brain is an obligatory consumer of glucose and thus is highly dependent on glycolysis.

However, glycolysis is always accompanied by the formation of glyoxal compounds, in particular methylglyoxal. These compounds are highly toxic as they easily form adducts with cellular proteins and nucleic acids and lead to their inactivation.

Therefore, all glycolyzing cells use detoxification systems which are mostly consisting of the enzymes glyoxalase I and II.

An alternative approach to therapeutic intervention aiming at influencing modified and increased glycolysis is therefore no longer related to the inhibition of glycolysis, but to the inhibition of glyoxalases, which has extensively been discussed in the context of cancer (Thornalley et al., 1994; Vander Jagt et al., 1990; Pemberton and Barrett, 1989; Creighton et al., 2003; Hamilton and Batist, 2004).

As pointed out above, bacteria, including pathogenic bacteria, perform glycolysis, which may be their main metabolic pathway for energy generation. Bacteria are classified as aerobic, anaerobic or facultative anaerobic. Aerobic and facultatively anaerobic bacteria can generate energy by different metabolic pathways, depending on the available level of oxygen. Under conditions of reduced oxygen, as is encountered e.g. in infected tissues, or within biofilms, such bacteria predominantly rely on glycolysis. Many anaerobic bacteria are exclusively dependent on glycolysis for energy generation from glucose. Except for a few exceptions all carbohydrate fermenting microorganisms under anaerobic conditions depend on energy gained by oxidizing glycerolaldehyde phosphate to pyruvate (glycolysis).

As pathogenic bacteria utilize glycolysis, as well as detoxifying glyoxalases, it is suggested by this invention that the inhibition of glycoxalases can serve as a "universal" therapy for a plurality of bacterial infections.

The term "bacterial infection" encompasses superficial colonisation by bacteria, e.g. of the skin, intraauricular or mucosa as well as systemic infections, including infections of blood, tissues and organs. Also encompassed are infections of the gastrointestinal tract. Compounds of the invention are used for the treatment of mucosal (topic) and/or systemic diseases. The mucosal diseases can be caused by oral or vaginal infections. The oral or vaginal infections are for example the consequence of AIDS, chemotherapy or an immune suppressive therapy or immune suppressive conditions.

The term "treatment" encompasses subjects suffering from any of the various disease stages, such as acute or chronic infection, and encompasses after-treatment as well as prophylaxis. "After-treatment" means a treatment following conventional therapy. Treatment concomitantly with conventional therapy (e.g. known antibiotics) is also part of the present invention.

The term "prophylaxis" or "chemo-preventivum" relates to administration of a pharmaceutical composition of the invention when a subject is at risk to develop a disease, or a disease is suspected or is present subclinically, but said disease has not fully evolved or has not been diagnosed.

The term "treating bacterial infections" in the stricter sense relates to the treatment of clinically manifest disease. It is meant to encompass both cytotoxic and cytostatic effects on bacterial cells. Thus, "inhibition of bacterial cells" encompasses the inhibition of cell proliferation (bacteristatic action) as well as the killing of the cells (bactericidal action). The killing of bacterial cells by necrosis or apoptosis is encompassed by the invention. The terms "proliferation" and "growth" are used interchangeably in the context of this application.

The present invention relates to the treatment and/or prophylaxis of bacterial infections by aerobic and/or anaerobic bacteria, gram positive and/or gram negative bacteria.

More specifically, the invention comprises, but is not limited to, the treatment of infections of one or more bacteria belonging to the genus Acrobacter, Actinobacillus, Actinomyces, Bacteroides, Borrelia, Bacillus, Brucella, Campylobacter, Clamydia, Clostridium, Corynebacterium, Cryptococcus, Enterobacter, Enterococcus, Erythrobacter, Eubacterium, Fusobacterium, gram-positiv cocci, Helicobacter, Hemophilus, Lactobacillus, Legionella, Listeria, Mycobacteria, Mycoplasma, Neissaria, Pasteurella, Peptostreptococcus, Pneumococcus, Porphyromonas, Prevotella, Pseudomonas, Rickettsia, Salmonella, Spirochetes (Borrelia, Treponema), Staphylococcus, Streptococcus, Vibrio, Yersinia, more specifically, Escherichia coli, Pneumocystis carinii, Helicobacter pylori or Borrelia burgdorferi.

In one embodiment, the invention is for the treatment of infections of bacteria belonging to one or more of the genus Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococus aureus and Fusobacterium nucleatum. The invention also relates to the treatment of periodontosis by one or more of the said bacteria.

The invention also relates to the treatment of bacterial infections comprising one or more selected from an airway infection, comprising upper and lower airway infections, skin infection, intraauricular infection, intestinal infection, gastric infection, systemic infection, comprising tissue and blood infections, or is parodontosis or infection due to implantation of medical devices comprising metal implants, metal joints, shunt tubing, vascular bypass grafts, contact lenses or is due to catheterization. In one embodiment the infection is a gastric infection caused by Helicobacter pylori, comprising chronic infection.

Importantly, the compounds of the present invention also affect bacteria that are resistant to conventional anti-bacterial therapy, such as penicillin resistant bacteria, because they act via a different mechanism.

Thus, in one embodiment of the invention, the bacterial infections are caused by a strain that is resistant to conventional anti bacterial agents, for example, one or more selected from the group comprising penicillins, aminoglycosides, tetracyclines, macrolide, fluoroquinolones, sulfonamides, vancomycin, trimethoprim, ciprofloxacin, oxazolidinones, linezolid, isoniazid, rifampin, methicillins and/or exhibit resistance due to expression of beta-lactamase, antibiotica transporter molecules, RNA methylation, and/or that resistance is due to other genetic changes of the bacteria

Importantly, the compounds of the present invention can also inhibit bacteria that are resistant against antibiotics, such as methicillin-resistant staphylococcus aureus (MRSA), which pose severe problems in the clinical setting (Cunha, 2005).

### b) Treatment of infections in immunosuppressed subjects

Bacterial infections represent a significant problem in patients in an immunosuppressed state, and are amongst the leading cause of death, e.g. in transplant patients.

Thus, in one embodiment of the invention, the infection is in an immunosuppressed animal, wherein said immunosuppression is associated with hereditary or acquired immune-defects, comprising acquired immune defect associated with HIV, organ transplantation, chemotherapy or exposure to radiation.

Infections on an immunsuppressed background are oftentimes opportunistic infections by organisms that are non-pathogenic in the normal individual. Treatment of opportunistic infections is encompassed by the present invention.

### c) Treatment of infections in cancer patients

Tumor patients are often in addition suffering from infectious diseases, due to a weakened immune defence, which results in a high sensitivity to infections.

Oftentimes cancer patients suffer from infectious disease caused by an ubiquitous, typically non-pathogenic organisms because of this weakened immune defence, also known as opportunistic infections.

It is therefore part of the invention to apply the substances, pharmaceutical composition or medicament of the invention for the treatment and/or prophylaxis against bacterial infections, comprising opportunistic infections, in cancer patients.

As it is known that most tumors show a high rate of glycolysis (Gatenby RA and Gillies RJ, 2004), the growth of tumor cells and infectious agents, such as bacteria, protozoa or fungi can be simultaneously inhibited, which represents an increase in efficacy for the treatment of such patients. Thus, it is a particular advantage of the present invention that cancer cells, like fungal, protozoal and bacterial cells, exhibit an increased glycolysis, accompanied by high activity of glyoxalases. Hence, cancer cells can be targeted by the substances of the invention, just like protozoal, fungal and bacterial cells. In other words, the substances of the invention at the same time inhibit both kinds of cells.

Glyoxalase I is up-regulated in many tumors. Generally, it is presumed that increasing concentrations of glyoxalase I correlate with the malignant phenotype of tumors. The increased concentration of glyoxalase I in tumor tissue in comparison to normal tissue is said to increase the resistance of tumors to chemotherapeutics like mitomycin C and other anti-cancer agents (Ranganathan et al., 1995; Ayoub et al., 1993). Inhibition of the glyoxalase I reaction by compounds of the present invention, such as ethyl pyruvate, alone or in combination with conventional cancer therapy, such as radiation or chemotherapy is therefore advantageous for the treatment of cancer.

According to the invention, the animal can be concomitantly suffering from cancer, and can be going to receive, is currently receiving, or has received conventional cancer therapy, comprising one or more of chemotherapy, surgery, radiotherapy or brachytherapy. It is meant to encompass treatment following a completed conventional therapy (e.g. a full regimen of chemotherapy comprising several individual treatment periods, or following surgery), and treatment that is intermittent with the conventional therapy, e.g. taking place in the intervals between individual courses of chemotherapy. It is also meant to relate to a therapy that is started after the conventional therapy (e.g. after the first course of chemotherapy) and then continues concomitantly with the first therapy (e.g. throughout the further courses of chemotherapy).

Advantageously, the substances of the invention also are effective in cancer cells that are resistant against conventional therapy, such as chemotherapy and/or radiation therapy.

Moreover, the known effect of substances of the invention, such as ethyl pyruvate as scavenger of reactive oxygen radicals represents a desired side effect for cells which do not have a high rate of glycolysis (non-cancer-cells) as such cells are additionally protected. In a combination therapy with a chemotherapeutic agent this is an additional advantage as also normal cells are stressed, which is reduced by compounds of the present invention.

In one embodiment the treatment of actinic keratoses with methyl- or ethylpyruvate is excluded.

The simultaneous inhibition of glyoxalases by compounds of the present invention such as ethyl or butyl pyruvate, ethyl or butyl lactate etc. in cancer cells as well as infectious organisms (in particular bacteria, fungi and protozoa) is particularly advantageous, as cancer cells and parasites are killed simultaneously.

### d) Treatment of concomitant infectious disease

Bacterial infections may be accompanied by other infections, such as fungal infections or protozoal infections. Such multiple infections are of particular significance in immune-compromised individuals, and pose a significant clinical problem in transplant patients, cancer patients or HIV patients.

For example, the weakening of the patient by cancer per se, as well as by cancer therapy, such as chemotherapy, which weakens the immune system, favors fungal as well as bacterial infections. For such patients, even ubiquitous bacteria that are non-pathogenic for healthy human beings can be dangerous (opportunistic infections), the more so pathogenic bacteria.

Degradation of glucose by glycolysis and the formation of glyoxal compounds are ubiquitous metabolic pathways, which are phylogenetically highly conserved (Heymans and Singh, 2003; Clugston et al., 1997). Cells which cover their energy consumption solely by glycolysis when glucose is available are for example fungal cells (e.g. Candida spp., Aspergillus spp., Cryptococcus spp., Zygomyces spp., Dermatophytes, Blastomyces spp., Histoplasma spp., Coccidoides spp., Sporothrix spp.), or cells of multicellular organisms like Helminthes (e.g. Schistosoma). They turn off other energy producing processes by glucose and use glycolysis (catabolite repression). It is therefore not surprising that many infectious organisms, such as fungi, bacteria and protozoa can also be inhibited in their growth by compounds of the present invention.

Similarly, pathogenic protozoa, such as the bloodstream forms of trypanosoma, leishmania, plasmodium or toxoplasma, but also bilharzia, depend exclusively on glucose as energy source and undergo glycolysis.

In one embodiment of the invention, therefore, the animal, including man, is concomitantly suffering from a fungal or protozoal infection as discussed above, including opportunistic infections and infections by organisms showing antibiotic resistance.

Specific examples of infectious organisms which can be treated according to the invention comprise Trypanosoma, Leishmania, Plasmodium, Toxoplasma, helmithes, Candida spp., Aspergillus spp., Cryptococcus spp., Pneumocystis spp., Zygomyces spp., Dermatophytes, Blastomyces spp., Histoplasma spp., Coccidoides spp., Sporothrix spp., Microsporidia spp., Malassezia spp. and Basidiomycetes.

Therefore, it is a particular advantage of the present invention that fungal infections as well as bacteria and protozoa can be effectively treated with the compounds of the present invention. Where in conventional therapies multiple active ingredients are required, the present invention provides substances that are effective against all these pathogens simultaneously. Consequently, the invention provides for a reduction in side effects, because only a single active ingredient is necessary, where the state of the art requires several different ingredients. In this context, the low toxicity of the compounds of the invention is a further particular advantage.

### e. "Postprandial state"

In one embodiment the pharmaceutical composition or medicament is for use in a vertebrate having a reduced blood glucose level. The use of the compounds of the present invention for the treatment in such a postprandial state is also part of the invention. In postprandial states the utilization of glucose is reduced in normal cells. These states can be reached for example by long-term fasting and can be accelerated by administration of hormones or can be forced by administration of hormones. Characteristic for such states is a low blood level of glucose and a high blood level of ketone bodies. Ketone bodies can be used by the brain to generate energy such that metabolic states of the patient can be generated under control of a medical practitioner prior to therapy wherein infectious organisms and/or tumors represent the primary consumers of glucose, under conditions of reduced blood glucose levels (Sugden and Holness, 2002).

Thus, in a postprandial state the selectivity of the therapy is enhanced, because of the reduced glucose metabolism in normal cells.

### D) Anti-bacterial applications

### a) Biofilms

The present invention relates to the prevention of the formation of biofilms, as well as combating biofilms by use of substances according to the invention.

More specifically, the invention relates to methods for preventing and/or combating biofilms, comprising contacting a substrate with a compound of the invention.

Bacteria can produce biofilms after attachment to a surface, which is a mixture of cells that coexist as an organized community. Biofilms represent a protective environment and thus biofilm formation carries important consequences, both in industrial and clinical settings.

Sessile bacterial cells within the biofilm are resistant to a range of antibacterial agents currently in clinical use. Biofilms can stick to plastic, are able to coat medical implants causing serious complications in patients with hip and valve replacements, shunt tubing weavers and contact lens weavers, vascular bypass crafts and urinary catheter (Reynolds TB, and Fink GR. Science 2001).

Microbes, such as bacteria, fungi, protozoa, or nematodes are found in dental unit waterlines. The presence of various microorganisms is a potential source of microbial contamination of dental aerosols, and thus a potential threat to the health of patients and dental staff. In particular under conditions of decreased immunity of an organism, opportunistic infections constitute a health risk (Szymanska J. Ann Agric Environ Med. 2005)

The presence of microbes in drinking water and within biofilms of water distribution systems are associated with taste and odor problems, contamination in food and beverage preparation, and a variety of health-related effects (Doggett MS. Appl Environ Microbiol. 2000).

Biofilms containing microbes can be dangerous in space stations. In long-term missions bacteria formation in closed systems can affect the astronauts health and water-thin biofilms can attack inaccessible cable harnesses causing electric breakdown.

Microorganisms have been implicated in the attack of both natural limestone materials and concrete. For example, the fungus Fusarium plays an important role in concrete deterioration. Treatment of microbial growth as constituents of biofilms is therefore promising for the protection of historical buildings and gravestones. (Gu, et al, International Biodeterioration & Biodegradation. 1998).

Microbial spoilage is a constant concern in the food processing industry. Even breweries have a risk of contamination of their products, even though there is a predisposition of beer per se to be contaminated with bacteria due to low pH-value (3.8-4.7). Most important beer-spoiling organisms belong to the genera Lactobacillus, Pediococus, Pectinatus, and to yeast representing Saccharomyces or Dekkera. Beer-spoiling bacteria are facultative or obligate anaerobes and are acidophilic or at least acidotolerent.

Biofilms have been found in brewery pasteurizers and conveyor systems. Bacteria associated with biofilms with conveyor tracks and bottles and can warmers belong to Pseudomonas, Enterobacter, Bacillus, and to yeast representing Saccharomyces, Candida, Rhodotorula and others (Storgards E, et al, J. Am. Soc. Brew. Chem. 2006).

Consequently, the present invention encompasses the combating of biofilms in all of the above settings, comprising contacting surfaces with substances of the invention to avoid biofilm formation and/or combat existing biofilms.

In one embodiment, the substance of the invention can be encompassed in a composition the substrate is covered with, e.g. a paint or coating layer. Such paint or coating layer may optionally result in the retarded liberation of the substances of the invention.

In the context of the present invention the term "biofilm" relates to bacteria attached to a substrate by means of an extracellular matrix produced by said bacteria, and comprising said bacteria. Biofilms can anchor bacteria to substrates formed by all kinds of materials e.g. metals, plastics, glass, concrete, limestone, soil particles, medical material and living substrates such as teeth. Thus, the invention relates to the prevention and or combating biofilms on such kinds of substrates.

In one embodiment, the substrate are teeth, and the biofilm may optionally be associated with the formation of dental plaque.

It is of particular advantage in this connection that substances of the invention not only affect bacteria, but also other organisms, such as e.g. fungi and protozoa. Biofilm are typically colonized by other organisms in addition to bacteria. Thus, the substances of the present invention can influence (i.e. inhibit their growth, and/or exert cytostatic and/or cytotoxic effects) many organisms typically found in biofilms (Armitage,2004; Leclerc, 2005; Coetser and Cloete, 2005; Chandra et al., 2005).

Thus, the invention relates to, but is not limited to, the prevention and combating of biofilm-formation in tube systems like pumps and filter systems (biofouling) for water distribution, conveyor tracks, bottles, can warmers in breweries, water tanks, clinical equipment like cathers, wound dressing material, colonisation of heat exchangers, paper machines, ship hulls, cooling water systems, oil recovery systems and corrosion of pipes (biocorrosion).

Moreover, the present invention relates to the treatment of infections associated with biofilms, such as infections caused by the inhalation of fragments of biofilms (e.g. inhaled biofilm fragments derived from contaminated inhalation devices, such as in dental unit waterlines or others).

It is a further particular advantage of the substances of the present invention that they can attack biofilms, which are difficult to combat with conventional antibiotics, such as colonisation of a cow's udder with biofilms.

Particular applications of the substances of the invention to combat bacteria, in particular in the context of biofilms, comprise the addition of said substances to fluid for storing and/or treating contact lenses, as an additive to or cleaning solution for dental unit waterlines, for use in the prevention of electric short circuits in electronic industry, for the cleaning of air ventilation systems by volatile substances of the invention, and/or their use as sprays/as a fog, their use in the protection of historical buildings and gravestones and monuments, as well as the use of the compounds of the invention to stabilize beer by preventing colonization with beer-spoiling organisms (e.g. by use of ethyl pyruvate or ethyl lactate, which degrades into ethanol and pyruvate/lactate) or to clean conveyor systems and bottles in breweries.

### b) Methods of treating drinking water

The present invention encompasses methods for treating water, in particular drinking water, with substances of the invention.

Drinking water is oftentimes contaminated by bacteria. As a matter of fact, many bacterial diseases, such as dysentery, are mainly transmitted by contaminated drinking water.

Thus, the present invention encompasses a method for treating drinking water, wherein water is contacted with substances of the invention. Such method comprises e.g. dosing of drinking water with such substances, or their use in filters and other devices or procedures for water purification. In a particular embodiment, the treatment is for the elimination of bacteria from the drinking water.

### c) General anti-bacterial applications

Moreover, the invention relates to contacting substrates with the substances of the invention as anti-bacterial agents, or incorporating the substances into compositions, wherein they act as anti-bacterial agents.

Thus, the compounds of the invention can serve as anti bacterial agents in all settings where antiseptic treatment is desired, and moreover serve as inhibitors of bacterial contamination of compositions, such as compositions containing organic substances. These include, but are not limited to, food and beverage compositions, non-food compositions, such as decorative paints, wallpaper paste: coating agents comprising paint; glues; and cleaning agents comprising household and industrial cleaning agents etc. In one embodiment the substances of the invention can be incorporated into conventional cleaning agents, comprising e.g. tensids. Such cleaning agents can be for domestic or industrial use, in particular for use in a clinical setting.

On the basis of the following figures and examples the present invention is illustrated further.

### BRIEF DESCRIPTION OF THE FIGURES

Fig. 1: Inhibition of the enzymatic activity of glyoxalase I of yeast by ethyl pyruvate (EP).
Fig. 2: Inhibition of the enzymatic activity of yeast glyoxalase I by 2-Oxopropanethioic acid S-ethyl ester (SE).
Fig. 3: Influence of ethyl pyruvate (EP), butyl pyruvate (BP) and butyl L-lactate (BL) on the enzymatic activity of yeast glyoxalase I.
Fig. 4: Influence of ethyl pyruvate (EP) on the enzymatic activity of yeast glyoxalase II.
Fig. 5: Inhibition of the enzymatic activity of glyoxalase I of human erythrocytes by ethyl pyruvate (EP).
Fig. 6: Inhibition of the enzymatic activity of glyoxalase II of human erythrocytes by ethyl pyruvate (EP).
Fig. 7: Effect of ethyl D-lactate (DEL), ethyl L-lactate (LEL) and ethyl pyruvate (EP) on the vitality of primary human fibroblasts.
Fig. 8: Effect of ethyl pyruvate (EP) on growth of different microbial species
Fig. 9: Effect of compounds of formula (I), (II), and (III) on anaerobic bacteria

### EXPERIMENTS

### A. General experimental procedures

### a) Determining enzymatic activity of yeast glyoxalase I

Measuring glyoxalase I (E.C.4.4.1.5, lactoyl-glutathione lyase Sigma, G-4252) was performed according to the instructions of McLellan and Thornalley (1989). The principle is based on measuring the initial rate of formation of S-D-lactoyl-glutathione from methylglyoxal (Sigma, M0252, 40%) and reduced L-glutathione (Aldrich, G-4251, >99%). The formation of the product is observed using the extinction coefficient e = 2,86 mM -1 cm -1 at 240 nm. The measurement was performed in 50 mM sodium phosphate buffer, pH 7,0. For that purpose 2 mM methylglyoxal and 2 mM reduced glutathione were incubated for 2 minutes at 30°C for the formation of the hemithioacetal. Thereafter 20 µl of a 1 to 1000 dilution of the glyoxalase I (Sigma, G-4252) was added to 1 ml of the measuring reagent to start the reaction.

Glyoxalase activity (IU) corresponds to the amount of enzyme forming 1 µmol of S-D-lactoyl-glutathione/min.

### b) Determining enzymatic activity of human erythrocyte glyoxalase I

The determination of glyoxalase I was performed as described above for the yeast enzyme. After the formation of the hemithioacetal, suitable amounts (5 to 100 µl) of an erythrocyte lysate were added to 1 ml of the measuring reagent to start the reaction. The erythrocyte lysate was prepared according to the instructions of Mannervik et al. (1982).

Glyoxalase activity (IU) corresponds to the amount of enzyme forming 1 µmol of S-D-lactoyl-glutathione/min.

### c) Determining enzymatic activity of human erythrocyte glyoxalase II

The determination of erythrocyte glyoxalase II (E.C.3.1.2.6., hydroxyacyl glutathione hydrolase) was performed according to the instructions of McLellan and Thornalley (1989). The principle is based on determining the initial hydrolysis of S-D-lactoyl-glutathione (L 7140, Sigma - Aldrich Chemie GmbH) in the presence of low amounts of a cell extract. Hydrolysis is observed on the basis of the reduction of the extinction at 240 nm (e = -3,1 mM -1 cm -1). Glyoxalase II activity (IU) correlates to the amount of enzyme hydrolysing 1 µmol of S-D-lactoyl-glutathione/min. The measurement was performed in 100 mM Tris-HCl buffer, pH 7,4. For this purpose 0.4 mM S-D-lactoyl-glutathione was added to 1 ml of the measuring reagent and the reaction was started by addition of suitable amounts (5-100 µl) of an erythrocyte hemolysate. The erythrocyte lysate was prepared according to the instructions of Mannervik et al. (1982).

### d) Protocol for experiments using LNCaP cells (androgen dependent prostate carcinoma cells; DSMZ No ACC 256)

An identical number of LNCaP cells (androgen dependent prostate carcinoma cells; DSMZ No ACC 256) were routinely cultured in 75 cm² culture flasks in RPMI-1640 medium (Gibco; Nr. 21875-034), penicillin/streptomycin (100 units penicillin/ml; 100 µg streptomycin/ml; Gibco; Nr. 15140/122) in the presence of 10% fetal calf serum (Biochrom; Nr. S0113/5; RPMI-FKS). The flasks were incubated at 37°C in a humidified atmosphere (relative humidity >95%) of 5% CO₂ in air. After reaching 50% confluency the medium was removed and the adherent cells were washed twice with PBS (phosphate buffered sodium chloride; 50 mM sodium phosphate, 150 mM NaCl, pH 7,4). Thereafter, the cells were incubated with serum free RPMI-medium (RPMI-SF) comprising the experimental supplements in five flasks each (i.e. five replicates each). The culture was continued at 37°C, 5% CO2 and 95% humidity for 24 hours. Thereafter the respective supernatants were removed and the adherent cells were detached from the bottom of the plate by trypsin/EDTA (Gibco; No. 25300-054) and were pelleted. After resuspension and homogenization the cells were counted using a hemocytometer.

### B. Data

### Example 1

Inhibition of the enzymatic activity of glyoxalase I of yeast by ethyl pyruvate (EP).

Determination of glyoxalase I activity was performed according to the general protocol as described above. The influence of ethyl pyruvate on enzyme activity was investigated by addition of increasing concentrations of EP (Sigma; no. E4,780-8; lot. S18972-513) to the measuring reagent.

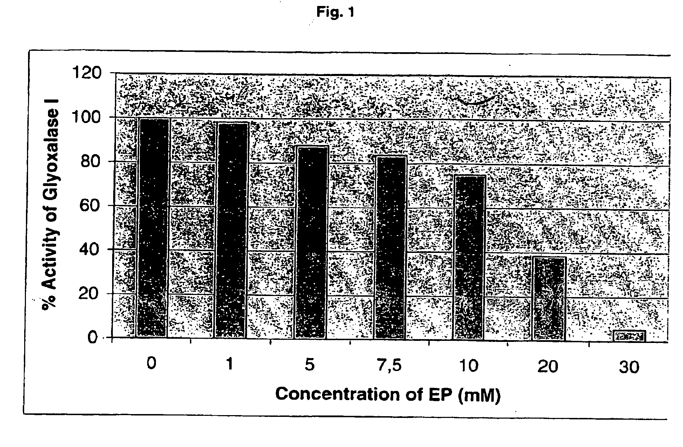

The experiments (Fig. 1) show that EP inhibits the reaction of yeast glyoxalase I in a concentration dependent manner.

### Example 2

Inhibition of yeast glyoxalase I by compounds of the general formula (I), (II), and (III), respectively

Determination of glyoxalase I activity was performed according to the general protocol as described above. The influence of compounds of the general formula (I), (II), and (III) on the activity of the enzyme was investigated by addition of increasing concentrations of these compounds to the preparation. The IC50-values were calculated from inhibition curves of each compound.

The data (Table 3) show that alkyl 2-oxo-derivatives inhibit the enzymatic activity of yeast glyoxalase I with different IC₅₀s, while the alkyl 2-hydroxy-derivatives revealed no inhibitory effect at all. Thus, the experiments demonstrate that alkyl 2-hydroxy-derivatives are prodrugs which must be activated to the respective 2-oxo-derivatives in living cells or organisms.

**Table 3**

| Alkyl 2-Oxo-Derivatives | | IC₅₀ GLOI* |
|---|---|---|
| Ethyl 2-oxobutyrate | CH3-CH2-O-CO-CO-CH2-CH3 | 1,8 mM |
| Butyl pyruvate | CH3-CH2-CH2-CH2-O-CO-CO-CH3 | 7,5 mM |
| Ethyl pyruvate | CH3-CH2-O-CO-CO-CH3 | 10 mM |
| Methyl pyruvate | CH3-O-CO-CO-CH3 | 15mM |
| Diethyl oxalate | CH3-CH2-O-CO-CO-OCH2-CH3 | >50mM |
| | | |

| Alkyl-2-Hydroxy Derivatives | | |
|---|---|---|
| (-)-Butyl L-Lactate | CH3-CH2-CH2-CH2-O-CO-CHOH-CH3 | no inhibition |
| (-)-Ethyl L-Lactate | CH3-CH2-O-CO-CHOH-CH3 | no inhibition |
| (+)-Ethyl D-Lactate | CH3-CH2-O-CO-CHOH-CH3 | no inhibition |
| Methyl L-Lactate | CH3-O-CO-CHOH-CH3 | no inhibition |
| (-)-isopropyl L-Lactate | CH3-CH(CH3)-O-CO-CHOH-CH3 | no inhibition |
| (+)-Isobutyl L-Lactate | CH3-CH(CH3)-CH2-O-CO-CHOH-CH3 | no inhibition |

| | | |
|---|---|---|
| *GLO I = glyoxalase I | | |

### Example 3

### Effect of prodrugs

The compounds of the general formula (II) and/or (III) can act as prodrug in the sense that the compounds are activated by enzymes within cells or in the organism, or are oxidized in vitro by addition of a suitable oxidant.

In cells or organisms lacking such activation systems the compounds of the general formula (II) and/or (III) remain inefficient. This is demonstrated by the effect of EP and LEL on the activity of glyoxalase I and the proliferation of tumor cells as well as yeast cells.

**Table 4**

| | Inhibition of enzyme | Inhibition of cell proliferation | |
|---|---|---|---|
| | yeast GLO I | LNCaP | Yeast cells |
| Ethyl pyruvate | + | + | + |
| Methyl L- | - | + | - |
| lactate | | | |

The experiment (Table 4) shows that alkyl 2-hydroxy derivatives have to be activated into alkyl 2-oxo-derivatives by endogenous activation systems to inhibit cell proliferation via inhibition of GLO1. In contrast to human tumor cells (LNCaP), yeast cells lack enzyme systems for transformation of alkyl 2-hydroxy derivatives into alkyl 2-oxo-derivatives and therefore proliferation can not be inhibited by alkyl 2-hydroxy derivatives directly.

### Example 4

Inhibition of the enzymatic activity of yeast glyoxalase I by 2-Oxopropanethioic acid S-ethyl ester (SE).

### Synthesis of 2-Oxopropanethioic acid S-ethyl ester

In a 500 ml three necked flask equipped with a reflux condenser and dropping funnel N, N'-dicyclohexylcarbodiimide (20.6 g, 0.1 mol; Cat.No. 36650, Lot. RA 13160, Fluka, Germany) was dissolved in dry tetrahydrofuran (200 mL; Cat.No. AE 07.1, Lot. 2121/SCR, Roth, Germany). Then, a solution of ethanethiol (6.2 g, 0.1 mol; Cat.No. EC 200-837-3, Lot. A0200018001, Acros Organics) in tetrahydrofuran (25 mL) was added. Under stirring a solution of 2-oxopropanoic acid (8.9 g, 0.1 mol) in tetrahydrofuran (25 mL) was added dropwise within 10 min without external heating. The solution warmed up to 45°C, turned yellow and a colourless precipitate of N,N'-dicyclohexylurea appeared. After complete addition the suspension was heated to reflux for 3 min and then cooled to 0° C. The urea was filtered off and the solvent removed from the filtrate by distillation. Without other manipulations the remaining orange oily residue was rapidly distilled in the air stream of a heat gun at normal pressure yielding a yellow fraction between boiling point (bp) 145 and 165 °C at normal pressure (amount of crude product 4.0 g, n_{D} 1.4802 (21 °C)) together with a considerable amount of brown resin in the distillation flask. The yellow crude product was redistilled in the same manner to yield 2.3 g of 2-oxopropanethioic acid S-ethyl ester as yellow oil, bp 153-157 °C, nD 1.4750 (21 °C), purity > 95 % (NMR).

1 H-NMR (300.06 MHz, CDCI3): □ 1.29 (-CH2-CH3), 2.41 (H3C-CO-), 2.92 (-CH2-CH3); 13C-NMR (75.45 MHz, CDCI3): □ 14.2 (-CH2-CH3), 23.2 (-CH2-CH3), 23.9 (H3C-CO-), 191.4 (-CO-), 193.4 (-CO-). NMR spectra were measured with a Varian Mercury-300BB spectrometer. Chemical shifts are reported at the □ scale in ppm.

### Determination of glyoxalase I activity

Determination of glyoxalase I activity was performed according to the general protocol as described above. The influence of 2-oxopropanethioic acid S-ethyl ester on the activity of the enzyme was investigated by addition of increasing concentrations of SE to the preparation for the measurement.

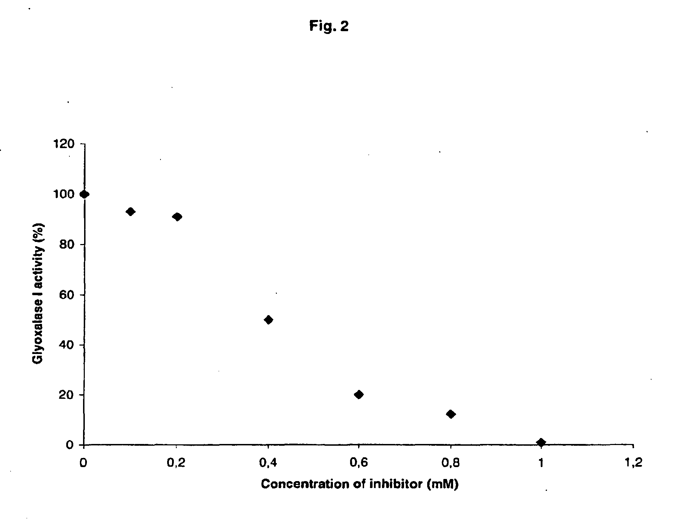

The experiment (Fig. 2) shows that SE inhibits the reaction of the glyoxalase I of yeast in a concentration dependent manner.

### Example 5

Influence of ethyl pyruvate (EP), butyl pyruvate (BP) and butyl L-lactate (BL) on the enzymatic activity of yeast glyoxalase I.

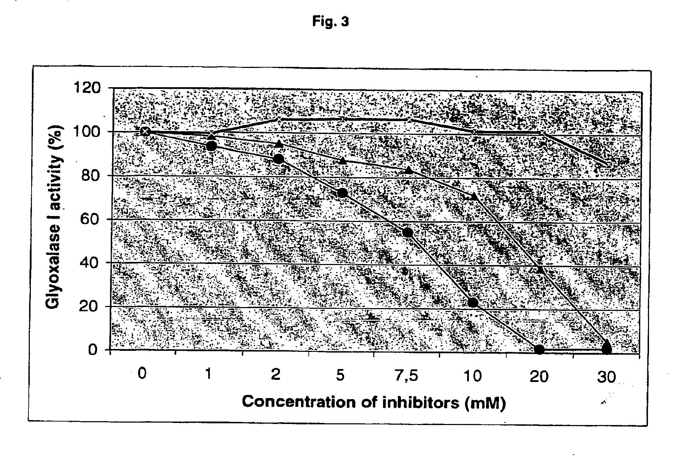

Determination of glyoxalase I activity was performed according to the general protocol as described above. The influence of effectors on the enzymatic activity was investigated by addition of increasing concentrations (0 - 30 mM) of EP (Sigma; no. E4,780-8; lot. S18972-513) (triangle), BP (prepared according to the instructions of patent JP 11080089; 98 %) (circle) and BL (Fluka, 69819; lot. 443090/1 21503090) (squares) to the measuring reagent (Fig. 3).

The experiments show that EP (triangles) as well as BP (circle) inhibit the activity of glyoxalase I in a concentration dependent manner wherein the effect of BP is stronger than the effect of EP. BL (squares) does not influence the reaction of glyoxalase when it is not transformed into BP.

### Example 6

Influence of ethyl pyruvate (EP) on the enzymatic activity of yeast glyoxalase II.

A colony of strain HD65-5a (Saccharomyces cerevisiae) was incubated in 5 ml YPD-medium [(2% glucose (Fluka), 1% yeast extract (BD, Sparks), 2% peptone (BD, Sparks)] over night at 30°C under rotation. A 10 ml aliquot was added to 200 ml culture medium in a 500 ml glass flask and was incubated at 30°C on a shaker (250 U/min). The yeast cells were harvested in the stationary growth face (O.D. 1cm/600nm = 2-4) by centrifugation (15 min, 3000 x g). The cells were diluted with 0.1 M MES buffer, pH 6,5 to an O.D. of 4 and were then disrupted in a glass mill (Schwock et al., 2004). Subsequently the disrupted cells were centrifuged at 23000 x g, 4°, 30 min. Protein concentration of the cell free extract was determined according to the Bradford method (Bradford, 1976).

The activity of the glyoxalase II (Hydroxyacyl glutathione hydrolase, E.C. 3.1.2.6.) was determined according to the instructions of Martins et al. (1999) in 0.1 M MES buffer, pH 6,5, 1.5 mM S-D-lactoyl-glutathione (L7140, Sigma-Aldrich Chemistry GmbH) and 0.75 mM DTNB (Sigma, D8130). After addition of suitable amounts of cell extract and an incubation period of 15 min at 25°C the formation of glutathione was measured at 412 nm (□ = 13.6 mM⁻¹ cm⁻¹). The activity of the glyoxalase II (IU) correlates with the amount of enzyme hydrolysing 1 µmol of S-D-lactoyl-glutathion/min.

The influence of ethyl pyruvate on the activity of the enzyme was investigated by adding increasing concentrations of EP (Sigma, No. E4,780-8; Lot. S18972-513) (0-20mM) to the measurement reagent.

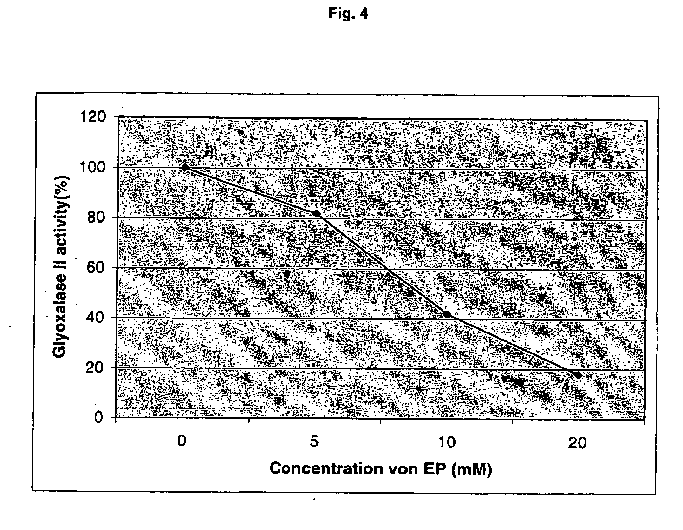

The relative activities of glyoxylase II in presence or absence of EP are illustrated in Fig. 4. The experiment shows that EP inhibits the reaction of yeast glyoxalase II in a concentration dependent manner.

### Example 7

Inhibition of the enzymatic activity of glyoxalase I of human erythrocytes by ethyl pyruvate (EP).

Determination of glyoxalase I activity was performed according to the general protocol as described above. The influence of ethyl pyruvate on enzyme activity was investigated by addition of increasing concentrations of EP (Sigma; no. E4,780-8; lot. S18972-513) (0-50mM) to the measuring reagent.

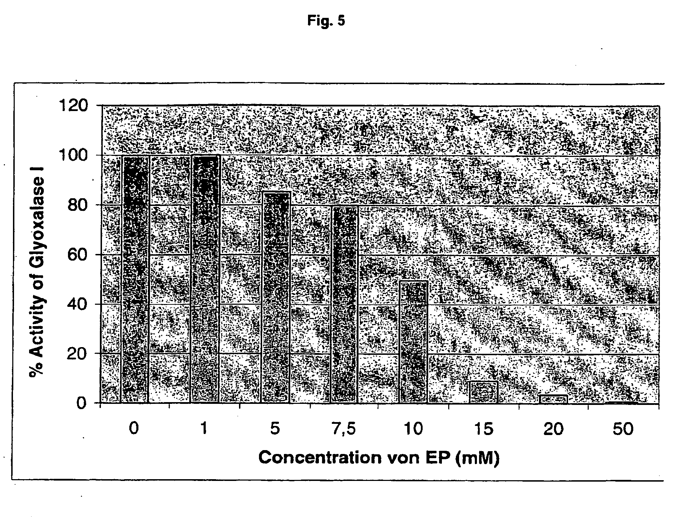

The experiment (Fig. 5) shows that EP inhibits the reaction of glyoxalase I of human erythrocytes in a concentration dependent manner.

### Example 8

Inhibition of the enzymatic activity of glyoxalase II of human erythrocytes by ethyl pyruvate (EP).

Determination of glyoxalase II activity was performed according to the general protocol as described above. The influence of ethyl pyruvate on the activity of the enzyme was investigated by addition of increasing concentrations of EP (Sigma, no. E 4,780-8; lot. S18972-513) (0-20 mM) to the measuring reagent.

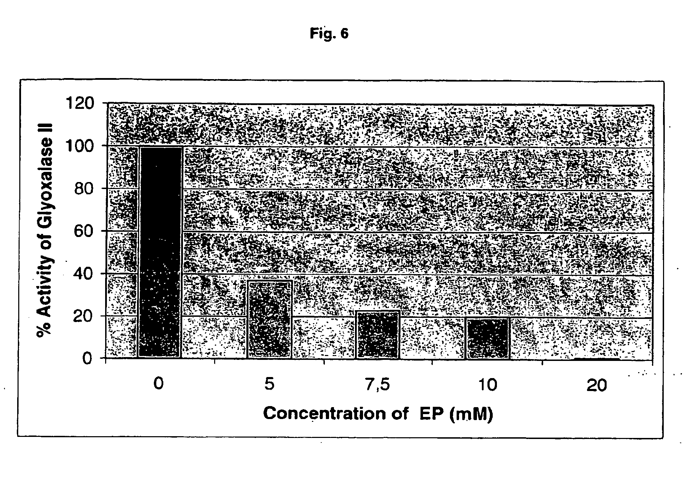

The experiment (Fig. 6) shows that EP inhibits the reaction of glyoxalase II in a concentration dependent manner.

### Example 9

Effect of ethyl D-lactate (DEL), ethyl L-lactate (LEL) and ethyl pyruvate (EP) on the vitality of primary human fibroblasts.

An identical number (104 cells per well) of primary human skin fibroblasts were inoculated in 24-well plates (Greiner, no. 662160) and were cultured in DMEM (Gibco; no. 41966-092) in the presence of 10 % calf serum (Biochrom, S0113/5), 2 mM L-glutamine (Gibco; no. 25030-024), penicillin/streptomycin (100 units penicillin/ml; 100 µg streptomycin/ml, Gibco; no. 15140/122), 5 mg% ascorbic acid (Serva, no. 14030.02) at 37°C, 5%CO2 and 95% humidity. The primary human fibroblasts were prepared according to the instructions of Birkenmeier et al. (1998). After reaching 50 % confluency the medium was by fresh serum free medium. Thereafter the following supplements were added to the cells: preparation 1 (equivalent volume of serum free (SF) medium, blank); preparation 2 (1 mM DEL or 1 mM LEL or 1 mM EP); preparation 3 (5 mM DEL or 5 mM LEL or 5 mM EP), preparation 4 (10 mM DEL or 10 mM LEL or 10 mM EP), preparation 5 (20 mM DEL or 20 mM LEL or 20 mM EP), preparation 6 (50 mM DEL or 50 mM LEL or 50 mM EP). The culture was continued at 37°C, 5% CO2 and 95% humidity for 24 hours. Thereafter the supernatants were removed and 100 µl of a 50 % thymol blue solution was added to the wells. After washing the cells with medium the unstained and stained cells were counted under the light optical microscope comprising a coordinate plane. Cells stained blue were assessed as avital, unstained cells as vital. The percentage of unstained cells of the total number of cells corresponds to the vitality of the cells.

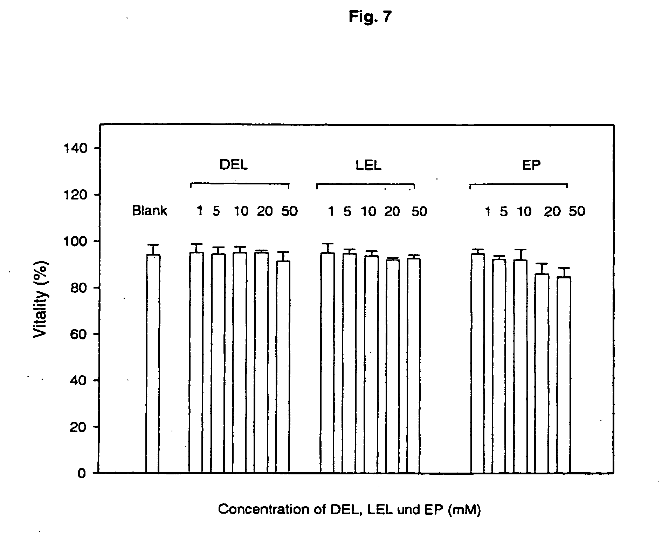

The experiment (Fig. 7) shows that DEL, LEL and EP are not toxic over the concentration range investigated and that they do not significantly influence vitality of primary human fibroblasts.

### Example 10

Effect of ethyl pyruvate (EP) on growth of different microbial species

The agar dilution method was used for antimicrobial susceptibility test. A series of plates (petri dishes; Greiner, CatNo. 933161 ) were prepared with 20 ml agar medium (Iso-Sensitest-Agar Ca.No. CM474, OXOID, Germany) to which various concentrations of EP (12.5mM to 40 mM) were added. Control agar plates were prepared without EP. The plates are then inoculated with a suitable standardized suspension of the test microbial species (10⁴ cfu) using an automated spotter (spots 1-21). The agar plates were incubated under aerobic conditions at 37°C for 20 hours. At the respective positions of the spots growth (if permissive under the selected conditions) will result in the formation of cell discs. The evaluation of the test results is performed by inspecting the sizes of these discs.

The following microbials were spotted to the agar dilution: (1) Candida albicans ATCC 90028 (2) Candida albicans (Patient isolate), (3) Candida krusei, ATCC 6258, (4) Candida krusei (Patient isolate), (5) Candida tropicalis (Patient isolate), (6) Candida glabrata (patient isolate), (7) Candida parapsilosis, ATCC 22019, (8) Escherichia coli, ATCC 25922, (9) Escherichia coli (Patient isolate) (10) Escherichia coli ESBL, (11) Klebsiella oxytoca ESBL, (12) Klebsiella oxytoca ESBL (Patient isolate); (13) Pseudomonas aeruginosa ATCC 27853, (14) Pseudomonas aeruginosa (Patient isolate), (15) Enterococcus faecalis ATCC 29212, (16) Enterococcus faecalis (Patient isolate), (17) Enterococcus faecium, (18) MRSA ATCC 43300, (19) MRSA (Patient isolate), (20) MSSA ATCC 25923, (21) MSSA (Patient isolate)

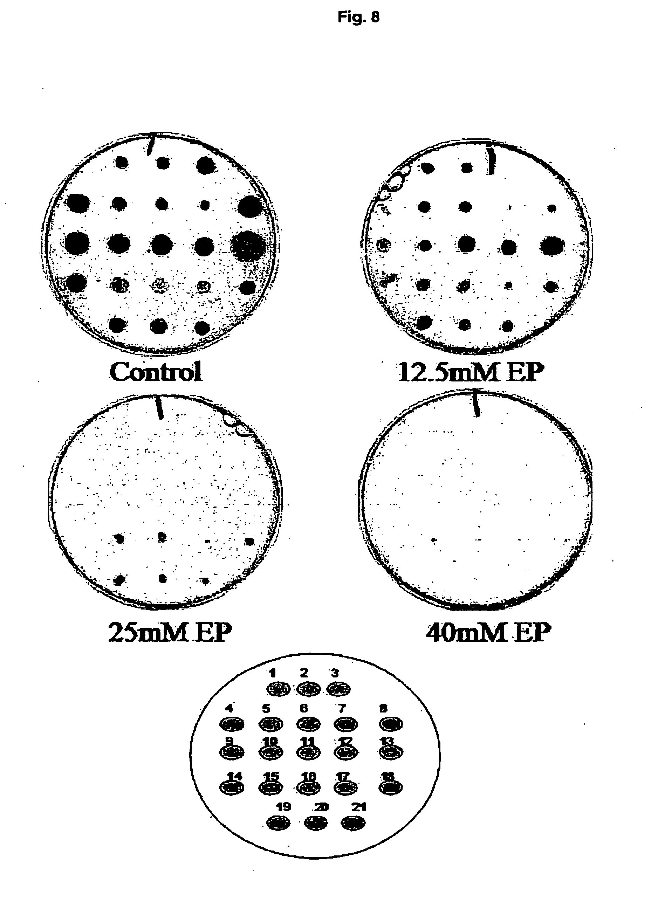

The experiment (Fig. 8) shows that EP effectively inhibits the growth and proliferation of different microbial species including fungi (1-7), gram negative bacteria (8-14), gram positive bacteria (15-21) among them even beta-lactamase positive bacteria as well as bacteria resistant to antibiotics such as methicillin-resistant Staphylococcus aureus (MRSA) species (18,19) (Cunha, 2005, Turner, 2005).

### Example 11

### Effect of compounds of formula (I), (II), and (III) on anaerobic bacteria

Antimicrobial susceptibility testing of compounds of formula (I), (II), and (III) was determined by agar dilution method. Brucella blood agar plates (20 ml) were prepared from Brucella agar (Becton Dickinson, CatNo. 211086) supplemented with hemin (50 g/ml), vitamin K (10 g/ml), and sterile defibrinated sheep blood (OXOID, Cat.No. SR0051C) according to (Claros et al., 1996). An inoculum for each isolate (bacteria) was prepared in the anaerobic chamber by harvesting 48 hours colony paste from brucella blood agar plates and suspending it in brucella broth [Brucella broth (Becton Dickinson) supplemented with hemin (50 µg/ml), vitamin K (10 g/ml), Sodium carbonate (0.1 mg/ml)] to a turbidity equivalent to a 0.5 McFarland Standard. The inocula were applied to agar plates containing compounds of invention with a replicator that delivers a final concentration of approximately 10⁵ cfu per spot. Plates containing no inventive compound were inoculated before and after each preparation of plates containing the compounds. Plates were incubated in an anaerobic chamber at 36°C for 48 hours and then read. The inhibitory concentrations of the invented compounds yielding a marked change in growth compared to control plates were judged.

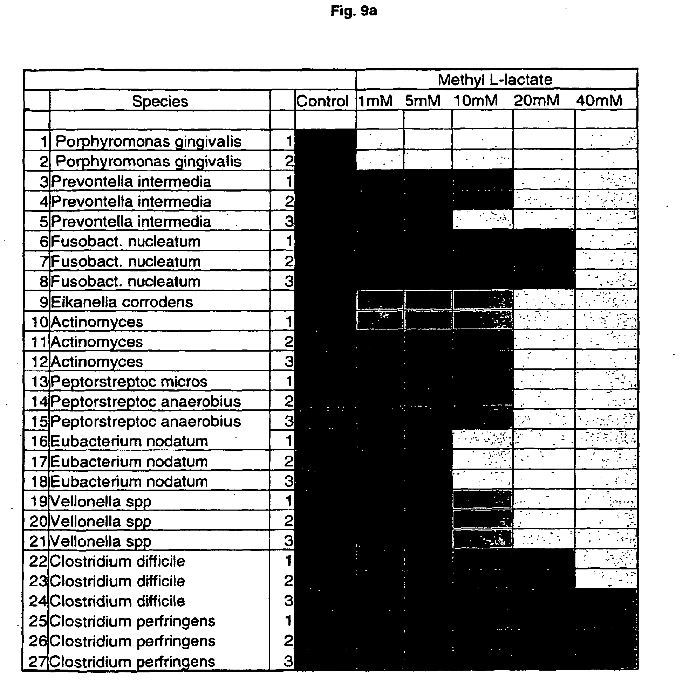

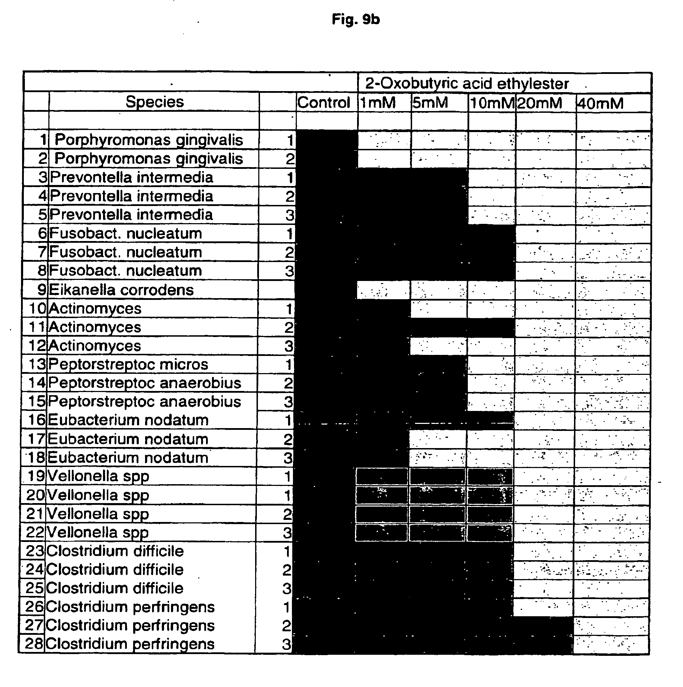

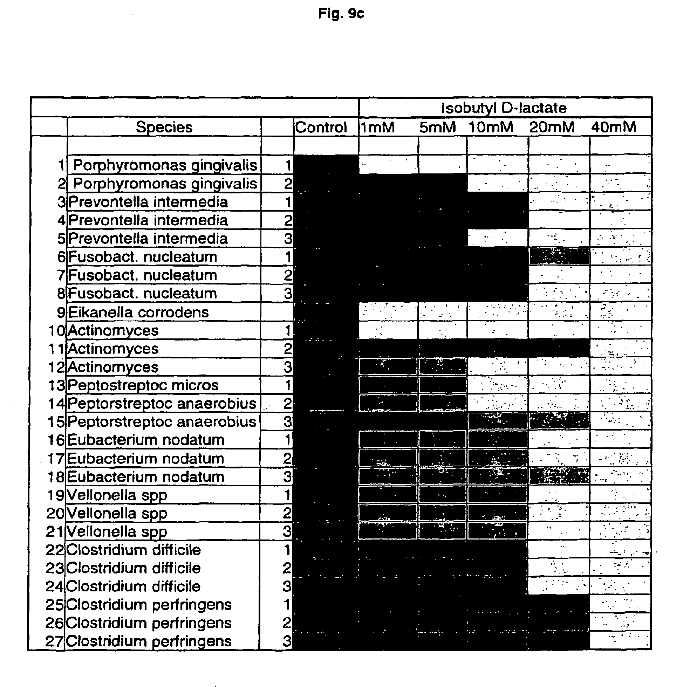

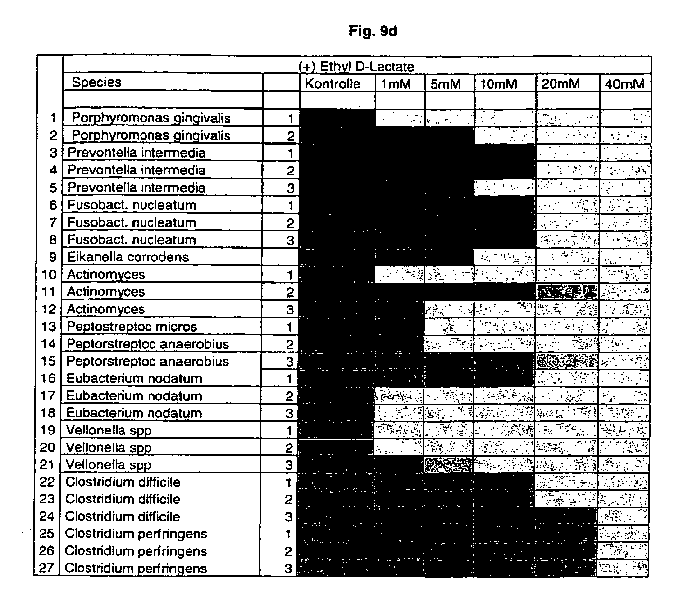

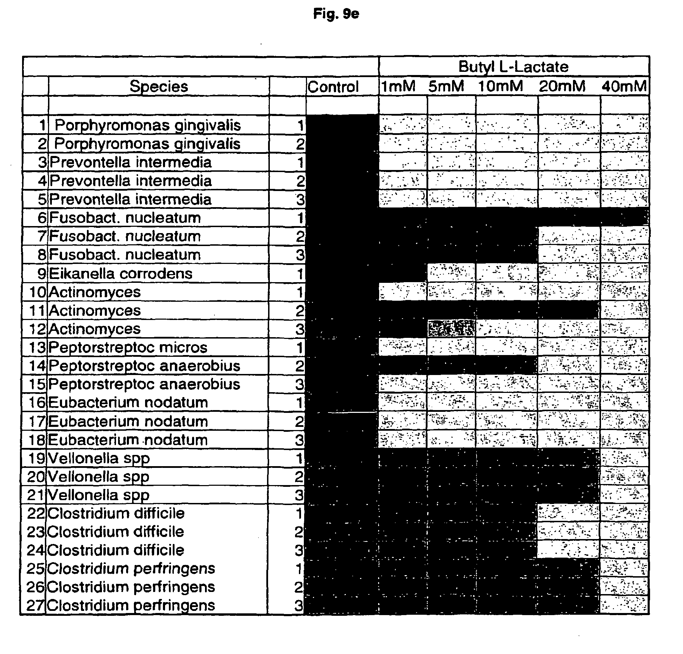

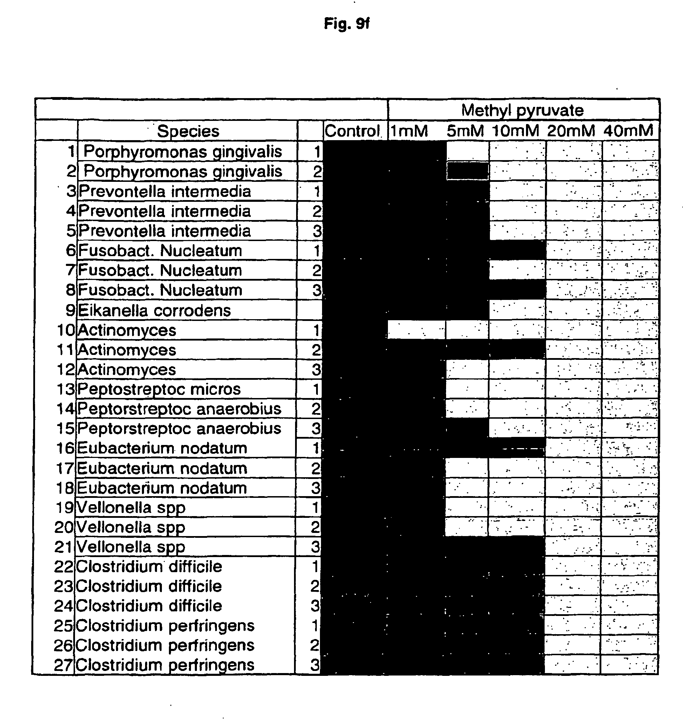

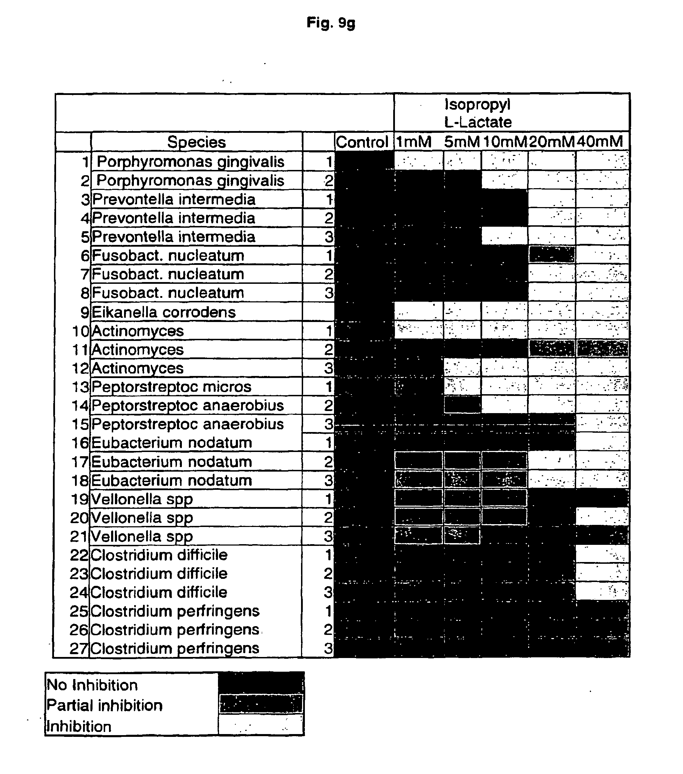

The experiment (Fig. 9a-h) shows that the growth of different anaerobic microbes can be inhibited by the invented compounds. In the figures, the numbers (1, 2, 3) refer to different isolates. Both, alkyl 2-hydroxy- as well as alkyl 2-oxo-derivatives show antimicrobial activity. In some bacteria the conversion of prodrug to active drug is probably impaired, which may lead to a reduced sensitivity.

### Example 12

### Destruction of biofilms on contaminated silicone surfaces

Biofilms were allowed to generate in a silicon tube system according to Gebel et al. (2005). In short, pipe water from a public water distributor was used as the source of biofilm-forming microorganisms. Water flow was at about 20 l per hour through silicon tubes from two commercial suppliers with inner diameters of 4 mm for 30 days at 25°C. After that the tubes were emptied, sealed at both ends and treated on their outer surface with 0.2 % peroxoacetic acid for 1 hour. Tubes were then rinsed with sterile water and afterwards cut into pieces of 5 cm length each using a sterile scalpel. Pieces obtained this way were then dipped into solutions which contained the substance to be tested or respective controls. 25 ml of the test solutions were used in closed Falcon tubes (50 ml). The tube segments were gently shaken for 30 min or 120 min, respectively, and then rinsed intensively with sterile water. Tubes were opened over their full length with scissors and the material deposited at the original inner surfaces of the tubes homogenized in 10 ml of saline (9 g NaCl/l water). Dilutions were made (decimal steps to 10⁴) and 0.1 ml of these solutions plated onto test agar plates (1 I contained 0.5 g yeast extract, 0.5 g peptone, 0.5 g casein hydrolysate, 0.5 g starch. 0.3 g potassium hydrogen phosphate, 24 mg magnesium sulphate, 0.3 g sodium pyruvate, 15 g agar). Plates were incubated for seven days at 20 °C. Colonies which formed were counted on each plate independent of their appearance and color. By experience, colonies of about at least 0.1 mm are detectable. Two dilutions must be consistent in colony numbers, taken into consideration the respective dilution factors. Numbers were normalized to 1 cm2 of the inner tube surface. Each substance was analysed in duplicates.

The experimental results given in Table 5 demonstrate the ability of ethyl pyruvate (EP) to destroy biofilms. The number of colony forming units decreases after treatment of biofilm with 25 mM EP from about 10⁷ to less then 10². An ester not encompassed by the general formula I is much less effective. EP is even more effective compared to a common disinfectant (peroxoacetic acid).

Biofilms were treated with water (control), ethyl pyruvate (EP), 25 mM ethyl acetate (ester control), or 0.1% peroxoacetic acid and the colony forming units determined as indicated. Numbers are given for each of the different silicon tubes used.

**Table 5: Colony forming units derivable from biofilms after treatment**

| | **Control** | | **Ester control** | | **10 mM EP** | | **25 mM EP** | | **Peroxo acetic acid** | |
|---|---|---|---|---|---|---|---|---|---|---|
| 30 min | 6.1 x10⁶ | 6.9 x10⁵ | 5.1 x10⁵ | 6.5 x10⁵ | 4.9 x10³ | 5.2 x10³ | 4.4 x10² | 4.2 x10² | 8.2 x10³ | 9.3 x10³ |
| 120 min | 5.9 x10⁶ | 6.3 x10⁶ | 7.0 x10⁴ | 1.1 x10⁵ | 1.6 x10³ | 1.7 x10³ | <10² | <10² | 3.1 x10³ | !4.7 x10³ |

### Example 13:

### Pharmaceutical composition for infusion

A solution for infusion comprising the substances of the invention is prepared as follows:
The compound of the invention, e.g. sterile ethyl pyruvate and/or ethyl lactate, is mixed with sterile 250 ml Lactated Ringers Balanced Salt Solution, pH 7.5, to achieve a final concentration of 0.05% to 10% per volume, e.g. 0.05%, 0.5%, 1%, 5%, or 10%, per volume. The pH of the solution is adjusted to 7.5 with NaOH, if necessary. After sterilization, the solution is packed in plastic containers and stored at 4°C. The composition of lactated Ringers Balanced Salt Solution is as follows:

| | |
|---|---|
| Sodium | 130 mM, |
| Calcium | 3,7 mM, |
| Potassium | 5,4 mM, |
| Chloride | 111,7 mM |
| Lactate | 27,2 mM. |

### Example 14:

### Pharmaceutical composition for bolus injection

A solution for bolus injection can be prepared according to Example 13, wherein the concentration of the substance of the invention is adapted accordingly.

### Example 15:

### Cream

A cream comprising a substance of the invention is prepared from the following ingredients:

| | | | |
|---|---|---|---|
| aqueous phase: | butyleneglycol | | 4% |
| | substance of the invention | 25% | |
| | water | | to 100% |
| lipid phase: | steareth-2 | 3% | |
| | steareth-21 | | 2% |
| | glycol-15-stearylether | 9% | |
| | cetearylalcohol | | 2,5% |
| therafter addition of: | | | |
| | phenoxyethanol, methylparaben, | | |
| | ethylparaben, propylparaben, | | |
| | butylparaben | 0,5% | |
| | butylenglycol | 0,5% | |
| | tocopherole | 0,2% | |

### Example 16:

### Ointment

An ointment of the oil-in-water-emulsion type, comprising a compound of the invention is prepared from the following ingredients.

| | | | | |
|---|---|---|---|---|
| A | product of the invention | 10-20% | | |
| | butyleneglycol | | 5% | |
| | glycerol | | | 4% |
| | sodium dihydroxy cetylphosphate, | | | |
| | isopropyl hydroxy cetylether | 2% | | |
| | water | | to 100% | |
| | | | | |
| B | glycolstearate SE | | 15% | |
| | octylcocoate | | 11% | |
| | | | | |
| C | butyleneglycol, metylparabene | | | |
| | ethylparabene, propylparabene, | | | |
| | pH: adjusted to 5,5 | | 2% | |

### Literature

Andersen PH, Jensen NJ. Mutagenic investigation of flavourings: dimethyl succinate, ethyl pyruvate and aconitic acid are negative in the Salmonella/mammalian-microsome test. Food Addit Contam. 1984 Jul-Sep;1(3):283-8
Armitage GC. Basic features of biofilms-why are they difficult therapeutic targets? Ann R Australas Coll Dent Surg. 2004 Oct;17:30-4. PMID: 16479852 [PubMed - in process]
Ayoub F, Zaman M, Thornalley P, Masters J. Glyoxalase activities in human tumour cell lines in vitro. Anticancer Res. 1993 Jan-Feb;13(1):151-5.
Bekeredjian R, Grayburn PA, Shohet RV. Use of ultrasound contrast agents for gene or drug delivery in cardiovascular medicine. J Am Coll Cardiol. 2005 Feb 1;45(3):329-35.
Bekeredjian R, Chen S, Frenkel PA, Grayburn PA, Shohet RV. Ultrasound-targeted microbubble destruction can repeatedly direct highly specific plasmid expression to the heart. Circulation. 2003 Aug 26;108(8):1022-6. Epub 2003 Aug 11.
Birkenmeier G, Heidrich K, Glaser C, Handschug K, Fabricius EM, Frank R, Reissig D. Different expression of the alpha2-macroglobulin receptor/low-density lipoprotein receptor-related protein in human keratinocytes and fibroblasts. Arch Dermatol Res. 1998 Oct;290(10):561-8.
Bowmer CT, Hooftman RN, Hanstveit AO, Venderbosch PW, van der Hoeven N. The ecotoxicity and the biodegradability of lactic acid, alkyl lactate esters and lactate salts. Chemosphere. 1998 Sep;37(7):1317-33.
Bradford M. M. A rapid and sensitive method for the quantitation of microgram quantities of protein utilizing the principle of protein-dye binding. Anal. Biochem. 1976, 72:248-254.
Brady RL, Cameron A. Structure-based approaches to the development of novel anti-malarials. Curr Drug Targets. 2004 Feb;5(2):137-49.
Clary JJ, Feron VJ, van Velthuijsen JA. Safety assessment of lactate esters. Regul Toxicol Pharmacol. 1998 Apr;27(2):88-97.
Clugston SL, Daub E, Kinach R, Miedema D, Barnard JF, Honek JF. Isolation and sequencing of a gene coding for glyoxalase I activity from Salmonella typhimurium and comparison with other glyoxalase I sequences. Gene. 1997 Feb 20;186(1):103-11.
Creighton DJ, Zheng ZB, Holewinski R, Hamilton DS, Eiseman JL. Glyoxalase I inhibitors in cancer chemotherapy. Biochem Soc Trans. 2003 Dec;31 (Pt 6):1378-82.
Chandra J, Zhou G, Ghannoum MA. Fungal biofilms and antimycotics. Curr Drug Targets. 2005 Dec;6(8):887-94. Review.
Coetser SE, Cloete TE. Biofouling and biocorrosion in industrial water systems. Crit Rev Microbiol. 2005;31(4):213-32. Review.
Cunha BA. Methicillin-resistant Staphylococcus aureus: clinical manifestations and antimicrobial therapy. Clin Microbiol Infect. 2005 Jui;11 Suppl 4:33-42.
Douglas KT, Gohel DI, Nadvi IN, Quilter AJ, Seddon AP. Partial transition-state inhibitors of glyoxalase I from human erythrocytes, yeast and rat liver. Biochim Biophys Acta. 1985 May 20;829(1):109-18.
Devamanoharan PS, Henein M, Ali AH, Varma SD. Attenuation of sugar cataract by ethyl pyruvate. Mol Cell Biochem. 1999 Oct;200(1-2):103-9.
Dong YQ, Yao YM, Wei P, Liu H, Dong N, Yu Y, Sheng ZY. Effects of ethyl pyruvate on cell-mediated immune function in rats with delayed resuscitation after burn injury. Zhongguo Wei Zhong Bing Ji Jiu Yi Xue. 2005 Jan;17(1):12-5.
Düfer M, Krippeit-Drews P, Buntinas L, Siemen D, Drews G. Methyl pyruvate stimulates pancreatic beta-cells by a direct effect on KATP channels, and not as a mitochondrial substrate. Biochem J. 2002 Dec 15;368(Pt 3):817-25.
Fan X, Subramaniam R, Weiss MF, Monnier VM. Methylglyoxal-bovine serum albumin stimulates tumor necrosis factor alpha secretion in RAW 264.7 cells through activation of mitogen-activating protein kinase, nuclear factor kappaB and intracellular reactive oxygen species formation. Arch Biochem Biophys. 2003 Jan 15;409(2):274-86.
Fink MP. Ethyl pyruvate: a novel treatment for sepsis and shock. Minerva Anestesiol. 2004 May;70(5):365-71.
Garcia CK, Goldstein JL, Pathak RK, Anderson RG, Brown MS. Molecular characterization of a membrane transporter for lactate, pyruvate, and other monocarboxylates: implications for the Cori cycle. Cell. 1994 Mar 11;76(5):865-73.
Gatenby RA, Gillies RJ. Why do cancers have high aerobic glycolysis? Nat Rev Cancer. 2004 Nov;4(11):891-9. Gebel, J., Otte, A., Exner, M. Wirksamkeitsprüfung biozider Wirkstoffe in biofilmkontaminierten Systemen unter praxisnahen Bedingungen. (2005) Hyg.Med. 30, 72-79.
Grant AW, Steel G, Waugh H, Ellis EM. A novel aldo-keto reductase from Escherichia coli can increase resistance to methylglyoxal toxicity. FEMS Microbiol Lett. 2003 Jan 21;218(1):93-9.
von Grumbckow L, Elsner P, Hellsten Y, Quistorff B, Juel C. Kinetics of lactate and pyruvate transport in cultured rat myotubes. Biochim Biophys Acta. 1999 Mar 4;1417(2):267-75. Hall SS, Doweyko LM, Doweyko AM, Zilenovski JS. Synthesis and evaluation of alpha-hydroxythiol esters as antitumor agents and glyoxalase I inhibitors. J Med Chem 1977 Oct. 20 (10): 1239-1242
Hamilton D, Batist G. Glutathione analogues in cancer treatment. Curr Oncol Rep. 2004 Mar;6(2):116-22.
Hamilton DS, Creighton DJ. Inhibition of glyoxalase I by the enediol mimic S-(N-hydroxy-N-methylcarbamoyl)glutathione. The possible basis of a tumor-selective anticancer strategy. J Biol Chem. 1992 Dec 15;267(35):24933-6.
Han Y, Englert JA, Yang R, Delude RL, Fink MP. Ethyl Pyruvate Inhibits Nuclear Factor-{kappa}B-Dependent Signaling by Directly Targeting p65. J Pharmacol Exp Ther. 2005 Mar;312(3):1097-105. Epub 2004 Nov 03.
Heymans M, Singh AK. Deriving phylogenetic trees from the similarity analysis of metabolic pathways. Bioinformatics. 2003;19 Suppl 1:i138-46.
Iwami Y, Schachtele CF, Yamada T. Mechansim of inhibition of Glycolysis in Streptococcus mutans NCIB 11723 by chlorhexidine. Oral Microbiol. Immunol. 1995 Dec; 10(6):360-4).
de Jaham C. Effects of an ethyl lactate shampoo in conjunction with a systemic antibiotic in the treatment of canine superficial bacterial pyoderma in an open-label, nonplacebo-controlled study. Vet Ther. 2003 Spring;4(1):94-100.
Johansson AS, Ridderstrom M, Mannervik B. The human glutathione transferase P1-1 specific inhibitor TER 117 designed for overcoming cytostatic-drug resistance is also a strong inhibitor of glyoxalase I. Mol Pharmacol. 2000 Mar;57(3):619-24.
Kalsi A, Kavarana MJ, Lu T, Whalen DL, Hamilton DS, Creighton DJ. Role of hydrophobic interactions in binding S-(N-aryl/alkyl-N hydroxycarbamoyl) glutathiones to the active site of the antitumor target enzyme glyoxalase I. J Med Chem. 2000 Oct 19;43(21):3981-6.
Kamiya D, Uchihata Y, Ichikawa E, Kato K, Umezawa K. Reversal of anticancer drug resistance by COTC based on intracellular glutathione and glyoxalase I. Bioorg Med Chem Lett. 2005 Feb 15;15(4):1111-4.
Kavanagh KL, Elling RA, Wilson DK. Structure of Toxoplasma gondii LDH1: active-site differences from human lactate dehydrogenases and the structural basis for efficient APAD+ use. Biochemistry. 2004 Feb 3;43(4):879-89.
N. Konietzko: "The combat against tuberculosis", pmi-publishing group 1996, ISBN: 3891193688)
Lakhdar-Ghazal F, Blonski C, Willson M, Michels P, Perie J. Glycolysis and proteases as targets for the design of new anti-trypanosome drugs. Curr Top Med Chem. 2002 May;2(5):439-56
Lembert N, Joos HC, Idahl LA, Ammon HP, Wahl MA. Methyl pyruvate initiates membrane depolarization and insulin release by metabolic factors other than ATP. Biochem J. 2001 Mar 1;354(Pt 2):345-50
Lens, P.; OFlaherty, V. [Ed.] Biofilms in Medicine, Industry and Environmental Biotechnology Characteristics, Analysis and Control IWA Publishing, 2003.
Leclerc H. Legionella: from environmental habitats to human disease] Bull Acad Natl Med. 2005 Jun;189(6):1221-33; discussion 1233-4. Review. French.
Lluis C, Bozal 4. [LDH and structural analogues of pyruvate (author's transl)] Rev Esp Fisiol. 1976 Mar;32(1):9-13
Mannervik B, Aronsson AC, Tibbelin G. Glyoxalase I from human erythrocytes. Methods Enzymol. 1982;90 Pt E:535-41.
Martins AM, Cordeiro C, Freire AP. Glyoxalase II in Saccharomyces cerevisiae: in situ kinetics using the 5,5'-dithiobis(2-nitrobenzoic acid) assay. Arch Biochem Biophys. 1999 Jun 1;366(1):15-20.
Marx E, Mueller-Klieser W, Vaupel P. Lactate-induced inhibition of tumor cell proliferation. Int J Radiat Oncol Biol Phys. 1988 May;14(5):947-55.
McLellan AC, Thornalley PJ. Glyoxalase activity in human red blood cells fractioned by age. Mech Ageing Dev. 1989 Apr;48(1):63-71
Miyaji T, Hu X, Yuen PS, Muramatsu Y, Iyer S, Hewitt SM, Star RA. Ethyl pyruvate decreases sepsis-induced acute renal failure and multiple organ damage in aged mice. Kidney Int. 2003 Nov;64(5):1620-31.
Mulier KE, Beilman GJ, Conroy MJ, Taylor JH, Skarda DE, Hammer BE. Ringer's ethyl pyruvate inhemorrhagic shock and resuscitation does not improve early hemodynamics or tissue energetics. Shock. 2005 Mar;23(3):248-252.
NCCLS National Committee for Clinical Laboratory Standards. Reference method for broth dilution antifungal susceptibility testing of yeast; approved standard. NCCLS document M27-A. Wayne, Pa, 1997 Nesterova M, Cho-Chung YS. Killing the messenger: antisense DNA and siRNA. Curr Drug Targets. 2004 Nov;5(8):683-9.
Pemberton KD, Barrett J. The detoxification of xenobiotic compounds by Onchocerca gutturosa (Nematoda: Filarioidea). Int J Parasitol. 1989 Dec;19(8):875-8.
Prottey C, George D, Leech RW, Black JG, Howes D, Vickers CF. The mode of action of ethyl lactate as a treatment for acne. Br J Dermatol. 1984 Apr;110(4):475-85.
Qin X, Weissman SJ, Chesnut MF, Zhang B, Shen L. Kirby-Bauer disc approximation to detect inducible third-generation cephalosporin resistance in Enterobacteriaceae. Ann Clin Microbiol Antimicrob. 2004 Jul 15;3(1):13.
Ranganathan S, Walsh ES, Tew KD. Glyoxalase I in detoxification: studies using a glyoxalase I transfectant cell line. Biochem J. 1995 Jul 1;309 (Pt 1):127-31.
Roth DA, Brooks GA. Lactate and pyruvate transport is dominated by a pH gradient-sensitive carrier in rat skeletal muscle sarcolemmal vesicles. Arch Biochem Biophys. 1990 Jun; 279(2):386-94.
Schwock J, Kirchberger J, Edelmann A, Kriegel TM, Kopperschlager G. Interaction of 6-phosphofructokinase with cytosolic proteins of Saccharomyces cerevisiae. Yeast. 2004 Apr 30;21(6):483-94.
Sharkey EM, O'Neill HB, Kavarana MJ, Wang H, Creighton DJ, Sentz DL, Eiseman JL. Pharmacokinetics and antitumor properties in tumor-bearing mice of an enediol analogue inhibitor of glyoxalase I. Cancer Chemother Pharmacol. 2000;46(2):156-66.
Stanko RT, Mullick P, Clarke MR, Contis LC, Janosky JE, Ramasastry SS. Pyruvate inhibits growth of mammary adenocarcinoma 13762 in rats. Cancer Res. 1994 Feb 15;54(4):1004-7.
Sugden MC, Holness MJ. Therapeutic potential of the mammalian pyruvate dehydrogenase kinases in the prevention of hyperglycaemia. Curr Drug Targets Immune Endocr Metabol Disord. 2002 Jul;2(2):151-65.
Thornalley PJ. The glyoxalase system in health and disease. Mol Aspects Med. 1993;14(4):287-371.
Thornalley PJ. Pharmacology of methylglyoxal: formation, modification of proteins and nucleic acids, and enzymatic detoxification--a role in pathogenesis and antiproliferative chemotherapy. Gen Pharmacol. 1996 Jun;27(4):565-73.
Thornalley PJ, Edwards LG, Kang Y, Wyatt C, Davies N, Ladan MJ, Double J. Antitumour activity of S-p-bromobenzylglutathione cyclopentyl diester in vitro and in vivo. Inhibition of glyoxalase I and induction of apoptosis. Biochem Pharmacol. 1996 May 17;51(10):1365-72.
Thornalley PJ, Strath M, Wilson RJ. Antimalarial activity in vitro of the glyoxalase I inhibitor diester, S-p-bromobenzylglutathione diethyl ester. Biochem Pharmacol. 1994 Jan 20;47(2):418-20.
Turner PJ. Extended-spectrum beta-lactamases. Clin Infect Dis. 2005 Aug 15;41 Suppl 4:S273-5.
Ulloa L, Ochani M, Yang H, Tanovic M, Halperin D, Yang R, Czura CJ, Fink MP, Tracey KJ. Ethyl pyruvate prevents lethality in mice with established lethal sepsis and systemic inflammation. Proc Natl Acad Sci USA. 2002 Sep 17;99(19):12351-6. Epub 2002 Sep 03
Valverde I, Cancelas J, Villanueva-Penacarrillo ML, Malaisse WJ. Potentiation by methyl pyruvate of GLP-1 insulinotropic action in normal rats. Int J Mol Med. 2001 Jun;7(6):621-3.
Vander Jagt DL, Hunsaker LA, Campos NM, Baack BR. D-lactate production in erythrocytes infected with Plasmodium falciparum. Mol Biochem Parasitol. 1990 Sep-Oct;42(2):277-84.
Varma SD, Devamanoharan PS, Ali AH. Prevention of intracellular oxidative stress to lens by pyruvate and its ester. Free Radic Res. 1998 Feb;28(2):131-5.
Vince R, Daluge S. Glyoxalase inhibitors. A possible approach to anticancer agents. J Med Chem. 1971 Jan;14(1):35-7.
Yang R, Uchiyama T, Watkins SK, Han X, Fink MP. Ethyl pyruvate reduces liver injury in a murine model of extrahepatic cholestasis. Shock. 2004 Oct;22(4):369-75.

### List of abbreviations

| | |
|---|---|
| ATP | Adenosine triphosphate |
| ATCC | American Type Culture Collection |
| BL | butyl lactate |
| BP | butyl pyruvate |
| DBL | butyl D-lactate |
| DEL | Ethyl D-lactate |
| DMEM | Dulbecco's modified Eagle Medium |
| DSMZ | German Collection of Microorganisms and Cell Cultures GmbH (Deutsche Sammlung von Mikroorganismen und Zellkulturen GmbH) |
| DTNB | 5,5'-dithiobis(2-nitro benzoic acid) |
| ECACC | European Collection of Cell Cultures |
| E.C. | Enzyme Commission |
| EDTA | ethylene diamine tetraacetate |
| EOB | ethyl -2-oxo-butyrate |
| EP | ethyl pyruvate |
| ESBL | expanded-spectrum beta-lactamase |
| FCS | fetal calf serum |
| GLOI | Glyoxalase I |
| HEPES | 4-(2-hydroxyethyl)-1-piperazineethanesulfonic acid |
| HIV | human immunodeficiency virus |
| IC-50 | Inhibitor conc. to reach 50% of the maximal effect |
| IU | international unit |
| LBL | Butyl L-lactate |
| LEL | Ethyl L-lactate |
| MES | 4-morpholine ethanesulfonic acid |
| MCT | monocarboxylate transporter |
| MIC | Minimal inhibition concentration |
| MRSA | Methicillin-resistant Staphylococcus Aureus |
| MSSA | Methicillin-sensitive Staphylococcus Aureus |
| NFkappaB | nuclear factor kappa B |
| O.D. | optical density |
| PBS | phosphate buffered saline |
| RPMI | Rosswell Park Memorial Institute |
| SF | serum free |
| YPD | yeast peptone dextrose medium |

## Claims

1. A substance for use in the treatment of bacterial infections in an animal, including man, according to the general formula (I) 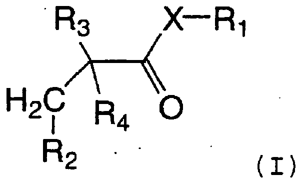 wherein
X is O or S; and
R1 is a branched or non-branched alkyl, cycloalkyl, branched or non-branched alkenyl, cycloalkenyl, branched or non-branched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =O,
or R3 is OH and R4 is H; or R3 is H and R4 is OH,
with the proviso that when X is O, R2 is H and R3 or R4 is OH, then R1 is not ethyl.

2. The substance according to claim 1, wherein R1 comprises 1 to 8 carbon atoms and R2 is H or comprises 1 to 8 carbon atoms.

3. The substance according to claim 1 or 2, wherein R1 comprises 1 to 4 carbon atoms and R2 is H or comprises 1 to 4 carbon atoms.

4. The substance according to any one of claims 1 to 3, wherein R1, R2 or R1 and R2 is methyl, ethyl, propyl, isopropyl, butyl, or isobutyl.

5. The substance according to any one of claims 1 to 4, wherein said substance is one or more selected from methyl pyruvate, ethyl pyruvate, isopropyl pyruvate, butyl pyruvate, isobutyl pyruvate, ethyl 2-oxobutyrate, butyl-2-oxobutyrate, cyclohexylmethyl pyruvate or the said compound wherein X = S

6. The substance according to any one of claims 1 to 4 wherein R3 or R4 is OH and it is selected from the group comprising the D-, L-enantiomer, and the racemic mixture thereof.

7. The substance according to any one of claims 1 to 6, wherein said bacteria comprise anaerobic bacteria and/or aerobic bacteria.

8. The substance according to any one of claims 1 to 7, wherein said bacteria comprise Gram positive and/or Gram negative bacteria.

9. The substance according to any one of claims 1 to 8, wherein the bacterial infection is associated with biofilms.

10. The substance according to claim 9, wherein said biofilm is formed on teeth.

11. The substance according to claim 10, wherein said biofilm is associated with the formation of dental plaque.

12. Use of a substance according to the general formula (I) 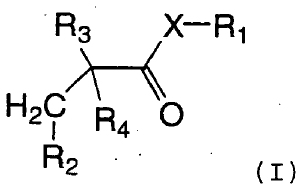 in the non-therapeutical prevention or combat of one or more selected from a biofilm and biocorrosion, wherein
X is O or S; and
R1 is a branched or non-branched alkyl, cycloalkyl, branched or non-branched alkenyl, cycloalkenyl, branched or non-branched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =O,
or R3 is OH and R4 is H; or R3 is H and R4 is OH,
with the proviso that when X is O, R2 is H and R3 or R4 is OH, then R1 is not ethyl.

13. The use according to claim 12, wherein said biofilm is formed on a support in an aquatic environment.

14. The use according to claim 12 or 13, wherein said support is one or more selected from metals, plastics, glass, concrete, limestone, soil particles, medical material, medical devices or tissues.

15. Method for preventing the formation of biofilms or for combating biofilms comprising contacting a substrate with at least one substance according to any one of claims 1 to 14, wherein the substrate is one or more selected from metals, plastics, glass, concrete, limestone and soil particles.

16. Pharmaceutical composition comprising at least one substance according to the general formula (I) 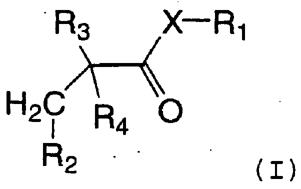 for use in the treatment of bacterial infections in an animal, including man, wherein X is O or S; and
R1 is a branched or non-branched alkyl, cycloalkyl, branched or non-branched alkenyl, cycloalkenyl, branched or nonbranched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =O; or R3 is OH and R4 is H; or R3 is H and R4 is OH with the proviso that when X=O, R2 is H and R3 or R4 is OH, then R1 is not ethyl.

17. The pharmaceutical composition according to claim 16, wherein R1 comprises 1 to 8 carbon atoms and R2 is H or comprises 1 to 8 carbon atoms.

18. The pharmaceutical composition according to claim 16, wherein R1 comprises 1 to 4 carbon atoms and R2 is H or comprises 1 to 4 carbon atoms.

19. The pharmaceutical composition according to any one of claims 17 to 18, wherein R1 and/or R2 is methyl, ethyl, propyl, isopropyl, butyl, or isobutyl.

20. The pharmaceutical composition according to any one of claims 17 to 19, wherein said substance is one or more selected from methyl pyruvate, ethyl pyruvate, propyl pyruvate, isopropyl pyruvate, butyl pyruvate, isobutyl pyruvate, ethyl 2-oxobutyrate, butyl-2-oxobutyrate, cyclohexylmethyl pyruvate or the said compounds wherein X = S.

21. The pharmaceutical composition according to any one of claims 17 to 19, wherein in said substance R3 or R4 is OH, and it is selected from the group comprising the D-, L-enantiomer and the racemic mixture thereof.

22. The pharmaceutical composition according to any one of claims 17 to 21, wherein said substance according to formula (I) is present in a therapeutically effective concentration.

23. The pharmaceutical composition according to any one of claims 17 to 22, which is for use in the treatment of bacterial infections associated with biofilms.

24. The pharmaceutical composition according to claim 23, wherein said biofilm is formed on a support in an aquatic environment.

25. The pharmaceutical composition according to claim 24, wherein said support is one or more selected from medical material, medical devices or tissues.

26. The pharmaceutical composition according to any one of claims 24 to 25, wherein said biofilm is associated with the formation of dental plaque.

27. The pharmaceutical composition according to any one of claims 17 to 26, which comprises one or more additional pharmaceutically active ingredients.

28. The pharmaceutical composition according to claim 27, wherein said additional pharmaceutically active ingredient comprises a compound which stimulates the metabolism of infectious organisms.

29. The pharmaceutical composition according to claim 28, wherein said compound which stimulates the metabolism of infectious organisms is glucose or 2,4-dinitrophenol.

30. The pharmaceutical composition according to claim 29, wherein said additional pharmaceutically active ingredient is selected from antibacterial agents.

31. The pharmaceutical composition according to claim 30, wherein said antibacterial agent is one or more selected from β-lactam antibiotics, azithromycin, aminoglycosides, tetracyclines, macrolide, quinolones and fluoroquinolones, sulfonamides, oxazolidones, biocidal peptides, trimethoprim, sulfamethoxazole, chloramphenicol, vancomycin, metronidazol and rifampin.

32. The pharmaceutical composition according to any one of claims 17 to 31, which further comprises one or more auxiliary substances, comprising fillers, flavouring agents and stabilizers.

33. The pharmaceutical composition according to any one of claims 17 to 32, wherein said composition is in the form of a sustained release or controlled release galenic formulation.

34. The pharmaceutical composition according to any one of claims 17 to 33, which is for topic or systemic administration.

35. The pharmaceutical composition according to any one of claims 17 to 34, which is for oral, intravenous, subcutaneous, intramuscular, intradermal, intraperitonal, intraauricular, rectal, intranasal, epidural, percutanous, transdermal or pulmonary administration, or for administration as an aerosol, oil, via mini-pumps, as mouth lavage, cream, ointment, spray, gel, plaster, and/or via microbubbles.

36. The pharmaceutical composition according to any one of claims 17 to 35, which is for use as a food supplement and/or beverage supplement.

37. The pharmaceutical composition according to any one of claims 17 to 36, wherein said animal is selected from invertebrates, non-mammalian vertebrates, and mammals including humans.

38. The pharmaceutical composition according to any one of claims 17 to 37, which is for use in the treatment and/or prophylaxis of bacterial infections resistant to antibiotic treatment.

39. The pharmaceutical composition according to claim 38, wherein said resistance is to one or more selected from the group comprising penicillins, aminoglycosides, tetracyclines, macrolide, fluoroquinolones, sulfonamides, vancomycin, trimethoprim, ciprofloxacin, oxazolidinones, linezolid, isoniazid, rifampin, methicillins.

40. The pharmaceutical composition according to any one of claims 17 to 39, which is for use in the treatment and/or prophylaxis of bacterial infections by aerobic and/or anaerobic bacteria.

41. The pharmaceutical composition according to any one of claims 17 to 40, which is for the treatment and/or prophylaxis of bacterial infections by gram positive and/or gram negative bacteria.

42. The pharmaceutical composition according to any one of claims 17 to 41, wherein said bacterial infection is one or more selected from an airway infection, comprising upper and lower airway infections, skin infection, intestinal infection, gastric infection, systemic infection, comprising tissue and blood infections, is parodontosis or infection due to implantation of medical devices comprising metal implants, metal joints or is due to catheterization.

43. The pharmaceutical composition according to any one of claims 17 to 42, which is for use in the treatment of infections of one or more bacteria belonging to the genus Acrobacter, Actinobacillus, Actinomyces, Bacteroides, Borrelia, Bacillus, Brucella, Campylobacter, Clamydia, Clostridium, Corynebacterium, Cryptococcus, Enterobacter, Enterococcus, Erythrobacter, Eubacterium, Fusobacterium, gram-positiv cocci, Helicobacter, Hemophilus, Lactobacillus, Legionella, Listeria, Mycobacteria, Mycoplasma, Neissaria, Pasteurella, Peptostreptococcus, Pneumococcus, Porphyromonas, Prevotella, Pseudomona, Pseudomonas, Rickettsia, Salmonella, Spirochetes Borrelia, Treponenia, Staphylococcus, Streptococcus, Vibrio, Yersinia, more specifically, Escherichia coli, Pneumocystis carinii, Helicobacter pylori or Borrelia burgdorferi and/or for use in the treatment of infections of bacteria belonging to one or more of the genus Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococus aureus and Fusobacterium nucleatum.

44. The pharmaceutical composition according to any one of claims 17 to 43, wherein said bacterial infection is an opportunistic infection.

45. The pharmaceutical composition according to any one of claims 17 to 44, which is for use in an immunosuppressed animal, including humans.

46. The pharmaceutical composition according to claim 45 wherein said immunosuppression is associated with hereditary or acquired immune-defects.

47. The pharmaceutical composition according to claim 46, wherein said acquired immune defect is associated with HIV, organ transplantation, chemotherapy or exposure to radiation.

48. The pharmaceutical composition according to any one of claims 17 to 47, which is for use in an animal, including man, concomitantly suffering from a fungal or protozoal infection.

49. The pharmaceutical composition according to any one of claims 17 to 48, which is for use in an animal, including man, having a reduced blood glucose level.

50. The pharmaceutical composition according to any one of claims 17 to 49, which is for use in an animal, including man, concomitantly suffering from cancer and/or wherein said animal, including man, is going to receive, is currently receiving, or has-received conventional cancer therapy.

51. The pharmaceutical composition according to claim 50, wherein said conventional cancer therapy is one or more of chemotherapy, surgery, radiotherapy or brachytherapy.

## Patentansprüche

1. Eine Substanz zur Verwendung in der Behandlung bakterieller Infektionen in einem Tier, den Menschen eingeschlossen, nach der allgemeinen Formel (I) 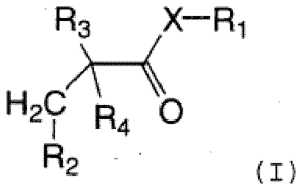 wobei gilt
X ist O oder S; und
R1 ist ein verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl, Aryl oder ein Zuckerrest; und
R2 ist H oder ein verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl oder Arylrest; und
R3 und R4 sind zusammen =O,
oder R3 ist OH und R4 ist H; oder R3 ist H und R4 ist OH,
unter der Bedingung, dass wenn X O ist, R2 H ist und R3 oder R4 OH ist, dann ist R1 nicht Ethyl.

2. Die Substanz nach Anspruch 1, wobei R1 ein bis acht Kohlenstoffatome enthält und R2 H ist oder ein bis acht Kohlenstoffatome enthält.

3. Die Substanz nach Anspruch 1 oder 2, wobei R1 ein bis vier Kohlenstoffatome enthält und R2 H ist oder ein bis vier Kohlenstoffatome enthält.

4. Die Substanz nach einem der Ansprüche 1 bis 3, wobei R1, R2 oder R1 und R2 Methyl, Ethyl, Propyl, Isopropyl, Butyl oder Isobutyl ist.

5. Die Substanz nach einem der Ansprüche 1 bis 4, wobei die benannte Substanz aus einer oder mehreren der folgenden Substanzen ausgewählt ist: Methyl-Pyruvat, Ethyl-Pyruvat, Isopropyl-Pyruvat, Butyl-Pyruvat, Isobutyl-Pyruvat, Ethyl-2-Oxobutyrat, Butyl-2-Oxobutyrat, Cyclohexylmethyl-Pyruvat oder die benannten Verbindungen mit X=S.

6. Die Substanz nach einem der Ansprüche 1 bis 4, wobei R3 oder R4 OH ist und die Substanz ausgewählt ist aus der Gruppe enthaltend das D-, L-Enantiomer, und das racemische Gemisch von diesen.

7. Die Substanz nach einem der Ansprüche 1 bis 6, wobei die genannten Bakterien anaerobe Bakterien und/oder aerobe Bakterien umfassen.

8. Die Substanz nach einem der Ansprüche 1 bis 7, wobei die genannten Bakterien Gram positive und/oder Gram negative Bakterien umfassen.

9. Die Substanz nach einem der Ansprüche 1 bis 8, wobei die bakterielle Infektion mit Biofilmen assoziiert ist.

10. Die Substanz nach Anspruch 9, wobei der genannte Biofilm auf Zähnen gebildet wird.

11. Die Substanz nach Anspruch 10, wobei der genannte Biofilm mit der Bildung von Plaque assoziiert ist.

12. Verwendung einer Substanz nach der allgemeinen Formel (I) 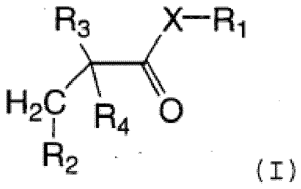 zur nicht-therapeutischen Vorbeugung oder Bekämpfung von einem oder mehreren ausgewählt aus einem Biofilm und Biokorrosion, wobei
X ist O oder S; und
R1 ist ein verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl, Aryl oder ein Zuckerrest; und
R2 ist H verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl oder Arylrest; und
R3 und R4 zusammen sind =O,
oder R3 ist OH und R4 ist H; oder R3 ist H und R4 ist OH,
unter der Bedingung, dass wenn X O ist, R2 H ist und R3 oder R4 OH ist, dann ist R1 nicht Ethyl.

13. Die Verwendung nach Anspruch 12, wobei der benannte Biofilm auf einem Träger in aquatischer Umgebung gebildet ist.

14. Die Verwendung nach Anspruch 12 oder 13, wobei der benannte Träger aus einer oder mehreren der folgenden ausgewählt ist: Metalle, Plastik, Glas, Beton, Kalkstein, Bodenteilchen, medizinische Materialien, Medizinprodukte oder Gewebe.

15. Methode zur Vorbeugung der Bildung von Biofilmen oder zur Bekämpfung von Biofilmen, umfassend das Inkontaktbringen eines Substrates mit mindestens einer Substanz nach einem der Ansprüche 1 bis 14, wobei das Substrat aus einem oder mehreren ausgewählt ist aus Metallen, Plastik, Glas, Beton, Kalkstein und Bodenpartikeln.

16. Pharmazeutische Zusammensetzung enthaltend mindestens eine Substanz nach der allgemeinen Formel (I) 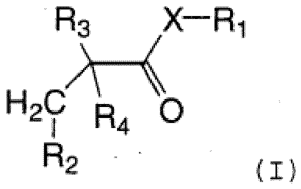 zur Verwendung in der Behandlung bakterieller Infektionen in einem Tier, den Menschen eingeschlossen, wobei
X ist O oder S; und
R1 ist ein verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl, Aryl oder ein Zuckerrest; und
R2 ist H verzweigtes oder unverzweigtes Alkyl, Cycloalkyl, verzweigtes oder unverzweigtes Alkenyl, Cycloalkenyl, verzweigtes oder unverzweigtes Alkinyl, Cycloalkinyl, Alkoxyalkyl, Alkoxycarbonylalkyl oder Arylrest; und
R3 und R4 zusammen sind =O; oder R3 ist OH und R4 ist H; oder R3 ist H und R4 ist OH
unter der Bedingung, dass wenn X O ist, R2 H ist und R3 oder R4 OH ist, dann ist R1 nicht Ethyl.

17. Die pharmazeutische Zusammensetzung nach Anspruch 16, wobei R1 ein bis acht Kohlenstoffatome enthält und R2 H ist oder ein bis acht Kohlenstoffatome enthält.

18. Die pharmazeutische Zusammensetzung nach Anspruch 16, wobei R1 ein bis vier Kohlenstoffatome enthält und R2 H ist oder ein bis vier Kohlenstoffatome enthält.

19. Die pharmazeutische Zusammensetzung nach einem der Ansprüche 17 bis 18, wobei R1 und/oder R2 Methyl, Ethyl, Propyl, Isopropyl, Butyl oder Isobutyl ist.

20. Die pharmazeutische Zusammensetzung nach einem der Ansprüche 17 bis 19, wobei die benannte Substanz aus einer oder mehreren der folgenden Substanzen ausgewählt ist: Methyl-Pyruvat, Ethyl-Pyruvat, Propyl-Pyruvat, Isopropyl-Pyruvat, Butyl-Pyruvat, Isobutyl-Pyruvat, Ethyl-2-Oxobutyrat, Butyl-2-Oxobutyrat, Cyclohexylmethyl-Pyruvat oder die benannten Verbindungen wobei X = S.

21. Die pharmazeutische Zusammensetzung nach einem der Ansprüche 17 bis 19, wobei in der benannten Substanz R3 oder R4 OH ist und welche ausgewählt ist aus der Gruppe enthaltend das D-, L-Enantiomer, und das racemische Gemisch davon.

22. Die pharmazeutische Zusammensetzung nach einem der Ansprüche 17 bis 21, wobei die benannte Substanz nach Formel (I) in einer therapeutisch effektiven Konzentration vorhanden ist.

23. Die pharmazeutische Zusammensetzung nach einem der Ansprüche 17 bis 22, zur Verwendung in der Behandlung von bakteriellen Infektionen, assoziiert mit Biofilmen.

24. Die pharmazeutische Zusammensetzung nach Anspruch 23, wobei der benannte Biofilm auf einem Träger in aquatischer Umgebung gebildet ist.

25. Die pharmazeutische Zusammensetzung nach Anspruch 24, wobei der benannte Träger aus einer oder mehreren ausgewählt ist aus medizinischen Materialien, Medizinprodukten oder Gewebe.

26. Die pharmazeutische Zusammensetzung nach einem der Ansprüche 24 bis 25, wobei der genannte Biofilm mit der Bildung von Plaque assoziiert ist.

27. Die pharmazeutische Zusammensetzung nach einem der Ansprüche 17 bis 26, enthaltend eine oder mehrere zusätzliche pharmazeutisch wirksame Inhaltsstoffe.

28. Die pharmazeutische Zusammensetzung nach Anspruch 27, wobei der benannte zusätzliche pharmazeutisch wirksame Inhaltsstoff eine Komponente enthält, welche den Metabolismus von infektiösen Organismen stimuliert.

29. Die pharmazeutische Zusammensetzung nach Anspruch 28, wobei die benannte Komponente, welche den Metabolismus von infektiösen Organismen stimuliert, Glukose oder 2,4-Dinitrophenol ist.

30. Die pharmazeutische Zusammensetzung nach Anspruch 29, wobei der benannte zusätzliche pharmazeutisch wirksame Inhaltsstoff aus antibakteriellen Agentien ausgewählt ist.

31. Die pharmazeutische Zusammensetzung nach Anspruch 30, wobei das benannte antibakterielle Agens aus einem oder mehreren der folgenden ausgewählt ist: β-Lactam-Antibiotika, Azithromycin, Aminoglycoside, Tetracycline, Makrolide, Quinolone und Fluoroquinolone, Sulfonamide, Oxazolidone, biozide Peptide, Trimethoprim, Sulfamethoxazol, Chloramphenicol, Vancomycin, Metronidazol und Rifampin.

32. Die pharmazeutische Zusammensetzung nach einem der Ansprüche 17 bis 31, weiterhin enthaltend einen oder mehrere Hilfsstoffe, umfassend Füllstoffe, Geschmacksstoffe und Stabilisatoren.

33. Die pharmazeutische Zusammensetzung nach einem der Ansprüche 17 bis 32, wobei die benannte Zusammensetzung in der Form einer galenischen Rezeptur mit andauernder Freisetzung oder kontrollierter Freisetzung ist.

34. Die pharmazeutische Zusammensetzung nach einem der Ansprüche 17 bis 33, für topische oder systemische Verabreichung.

35. Die pharmazeutische Zusammensetzung nach einem der Ansprüche 17 bis 34, zur oralen, intravenösen, subkutanen, intramuskulären, intradermalen, intraperitonalen, intraaurikulären, rektalen, intranasalen, epiduralen, perkutanen, transdermalen oder pulmonalen Anwendung oder zur Anwendung als ein Aerosol, Öl, mittels Mini-Pumpen, als Mundspülung, Creme, Salbe, Spray, Gel, Pflaster und/oder über Mikrobläschen.

36. Die pharmazeutische Zusammensetzung nach einem der Ansprüche 17 bis 35, zur Verwendung als Nahrungsergänzungsmittel und/oder Getränke-Ergänzungsmittel.

37. Die pharmazeutische Zusammensetzung nach einem der Ansprüche 17 bis 36, wobei das genannte Tier ausgewählt ist aus Wirbellosen, nicht-säugenden Wirbeltieren und Säugetieren einschließlich dem Menschen.

38. Die pharmazeutische Zusammensetzung nach einem der Ansprüche 17 bis 37, zur Verwendung in der Behandlung und/oder Prophylaxe von bakteriellen Infektionen, die resistent gegenüber antibiotischer Behandlung sind.

39. Die pharmazeutische Zusammensetzung nach Anspruch 38, wobei die benannte Resistenz gegenüber einem oder mehreren Stoffen ausgewählt aus der Gruppe umfassend Penizilline, Aminoglycoside, Tetracycline, Makrolide, Fluoroquinolone, Sulfonamide, Vancomycin, Trimethoprim, Ciprofloxacin, Oxazolidinone, Linezolid, Isoniazid, Rifampin, Methicilline.

40. Die pharmazeutische Zusammensetzung nach einem der Ansprüche 17 bis 39, zur Verwendung in der Behandlung und/oder Prophylaxe von bakteriellen Infektionen mit aerobischen und/oder anaerobischen Bakterien.

41. Die pharmazeutische Zusammensetzung nach einem der Ansprüche 17 bis 40, zur Verwendung in der Behandlung und/oder Prophylaxe von bakteriellen Infektionen mit Gram positiven und/oder Gram negativen Bakterien.

42. Die pharmazeutische Zusammensetzung nach einem der Ansprüche 17 bis 41, wobei die benannte bakterielle Infektion aus einer oder mehreren der folgenden ausgewählt ist: Atemwegsinfektion, umfassend obere und untere Atemwegsinfektionen, Hautinfektion, intestinale Infektion, gastrische Infektion, systemische Infektion, beinhaltend Gewebs- und Blutinfektionen, Parodontitis oder Infektion aufgrund von Implantation von medizinischen Hilfsmitteln, umfassend Metallimplantate, Metallgelenke, oder aufgrund von Katheterisierung.

43. Die pharmazeutische Zusammensetzung nach einem der Ansprüche 17 bis 42, zur Verwendung in der Behandlung von Infektionen durch eine oder mehrere Bakterien der Gattung Acrobacter, Actinobacillus, Actinomyces, Bacteroides, Borrelia, Bacillus, Brucella, Campylobacter, Clamydia, Clostridium, Corynebacterium, Cryptococcus, Enterobacter, Enterococcus, Erythrobacter, Eubacterium, Fusobacterium, Grampositive Koggen, Heliobacter, Hemophilus, Lactobacillus, Legionella, Listeria, Mycobacteria, Mycoplasma, Neissaria, Pasteurella, Peptostreptococcus, Pneumococcus, Porphyromonas, Prevotella, Pseudomona, Pseudomonas,
Rickettsia, Salmonella, Spirochaetes borellia, Treponema, Staphylococcus, Streptococcus, Vibrio, Yersenia, bevorzugt Escherichia coli, Pneumocystis carinii, Heliobacter pylori oder Borrelia burgdorferi und/oder zur Verwendung in der Behandlung von Infektionen durch Bakterien ausgewählt aus einer oder mehreren der Gattungen Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococcus aureus und Fusobacterium nucleatum.

44. Die pharmazeutische Zusammensetzung nach einem der Ansprüche 17 bis 43, wobei die besagte bakterielle Infektion eine opportunistische Infektion ist.

45. Die pharmazeutische Zusammensetzung nach einem der Ansprüche 17 bis 44, zur Verwendung in einem immunsupprimierten Tier, einschließlich dem Menschen.

46. Die pharmazeutische Zusammensetzung nach Anspruch 45, wobei die benannte Immunsupression assoziiert ist mit vererbten oder erworbenen Immundefekten.

47. Die pharmazeutische Zusammensetzung nach Anspruch 46, wobei der benannte erworbene Immundefekt assoziiert ist mit HIV, Organtransplantation, Chemotherapie oder Belastung durch Strahlung.

48. Die pharmazeutische Zusammensetzung nach einem der Ansprüche 17 bis 47, zur Verwendung in einem Tier, den Menschen eingeschlossen, welches begleitend an Pilz- oder Protozoen-Infektion leiden.

49. Die pharmazeutische Zusammensetzung nach einem der Ansprüche 17 bis 48, zur Verwendung in einem Tier, den Menschen eingeschlossen, mit verringertem Blutzuckerspiegel.

50. Die pharmazeutische Zusammensetzung nach einem der Ansprüche 17 bis 49, zur Verwendung in einem Tier, den Menschen eingeschlossen, welche begleitend an Krebs leiden oder wobei besagtes Tier, den Menschen eingeschlossen, eine konventionelle Krebstherapie erhalten wird, erhält oder erhalten hat.

51. Die pharmazeutische Zusammensetzung nach Anspruch 50, wobei die benannte konventionelle Krebstherapie aus einer oder mehrerer der folgenden ausgewählt ist: Chemotherapie, Operation, Radiotherapie oder Brachytherapie.

## Revendications

1. Substance à utiliser dans le traitement d'infections bactériennes chez un animal, notamment un homme, correspondant à la formule générale (I) 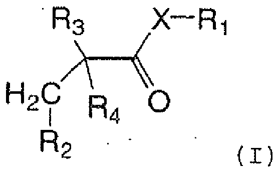 dans laquelle
X est O ou S ; et
R1 est un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle, aryle, ou un résidu sucre ; et
R2 est H ou un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle ou aryle ; et
R3 et R4 pris ensemble sont = O,
ou R3 est OH et R4 est H ; ou R3 est H et R4 est OH,
à la condition que lorsque X est O, R2 est H et R3 ou R4 est OH, alors R1 n'est pas un groupe éthyle.

2. Substance selon la revendication 1, dans laquelle R1 comprend 1 à 8 atomes de carbone et R2 est H ou comprend 1 à 8 atomes de carbone.

3. Substance selon la revendication 1 ou 2, dans laquelle R1 comprend 1 à 4 atomes de carbone et R2 est H ou comprend 1 à 4 atomes de carbone.

4. Substance selon l'une quelconque des revendications 1 à 3, dans laquelle R1, R2 ou R1 et R2 est/sont un groupe méthyle, éthyle, propyle, isopropyle, butyle ou isobutyle.

5. Substance selon l'une quelconque des revendications 1 à 4, dans laquelle ladite substance est un ou plusieurs des éléments sélectionnés parmi le pyruvate de méthyle, le pyruvate d'éthyle, le pyruvate d'isopropyle, le pyruvate de butyle, le pyruvate d'isobutyle, le 2-oxobutyrate d'éthyle, le 2-oxobutyrate de butyle, le pyruvate de cyclohexylméthyle ou ledit composé dans lequel X = S.

6. Substance selon l'une quelconque des revendications 1 à 4 dans laquelle R3 ou R4 est OH, qui est sélectionnée dans le groupe composé du D-énantiomère ou du L-énantiomère et du mélange racémique de ceux-ci.

7. Substance selon l'une quelconque des revendications 1 à 6, dans laquelle ladite bactérie comprend une bactérie anaérobie et/ou une bactérie aérobie.

8. Substance selon l'une quelconque des revendications 1 à 7, dans laquelle ladite bactérie comprend une bactérie Gram positive et/ou Gram négative.

9. Substance selon l'une quelconque des revendications 1 à 8, dans laquelle ladite infection bactérienne est associée à des biofilms.

10. Substance selon la revendication 9, dans laquelle ledit biofilm est formé sur les dents.

11. Substance selon la revendication 10, dans laquelle ledit biofilm est associé à la formation de la plaque dentaire.

12. Utilisation d'une substance selon la formule générale (I) 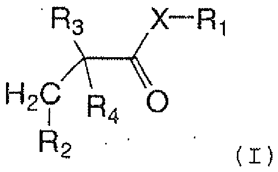 dans la prévention non thérapeutique ou le combat d'un ou de plusieurs éléments sélectionnés parmi un biofilm et une corrosion biologique, où X est O ou S ; et
R1 est un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle, aryle ou un résidu sucre ; et
R2 est H ou un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle ou aryle ; et
R3 et R4 pris ensemble sont = O,
ou R3 est OH et R4 est H ; ou R3 est H et R4 est OH,
à la condition que lorsque X est O, R2 est H et R3 ou R4 est OH, alors R1 n'est pas un groupe éthyle.

13. Utilisation selon la revendication 12, dans laquelle ledit biofilm est formé sur un support dans un environnement aquatique.

14. Utilisation selon la revendication 12 ou 13, dans laquelle ledit support est un ou plusieurs éléments sélectionnés parmi des métaux, des plastiques, du verre, du béton, du calcaire, des particules du sol, un matériel médical, des dispositifs ou des tissus médicaux.

15. Méthode de prévention de la formation de biofilms ou de lutte contre des biofilms comprenant la mise en contact d'un substrat avec au moins une substance selon l'une quelconque des revendications 1 à 14, où le substrat est un ou plusieurs éléments sélectionnés parmi des métaux, des plastiques, du verre, du béton, du calcaire, des particules du sol.

16. Composition pharmaceutique comprenant au moins une substance correspondant à la formule générale (I) 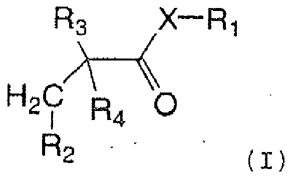 à utiliser dans le traitement d'infections bactériennes chez un animal, notamment un homme, où
X est O ou S ; et
R1 est un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle, aryle, ou un résidu sucre ; et
R2 est H ou un groupe alkyle ramifié ou non ramifié, cycloalkyle, alcényle ramifié ou non ramifié, cycloalcényle, alcynyle ramifié ou non ramifié, cycloalcynyle, alcoxyalkyle, alcoxycarbonylalkyle, ou aryle ; et
R3 et R4 pris ensemble sont = O ; ou R3 est OH et R4 est H ; ou R3 est H et R4 est OH,
à la condition que lorsque X est O, R2 est H et R3 ou R4 est OH, alors R1 n'est pas un groupe éthyle.

17. Composition pharmaceutique selon la revendication 16, dans laquelle R1 comprend 1 à 8 atomes de carbone et R2 est H ou comprend 1 à 8 atomes de carbone.

18. Composition pharmaceutique selon la revendication 16, dans laquelle R1 comprend 1 à 4 atomes de carbone et R2 est H ou comprend 1 à 4 atomes de carbone.

19. Composition pharmaceutique selon l'une quelconque des revendications 17 à 18, dans laquelle R1 et/ou R2 est/sont un groupe méthyle, éthyle, propyle, isopropyle, butyle ou isobutyle.

20. Composition pharmaceutique selon l'une quelconque des revendications 17 à 19, dans laquelle ladite substance est un ou plusieurs éléments sélectionnés parmi le pyruvate de méthyle, le pyruvate d'éthyle, le pyruvate de propyle, le pyruvate d'isopropyle, le pyruvate de butyle, le pyruvate d'isobutyle, le 2-oxobutyrate d'éthyle, le 2-oxobutyrate de butyle, le pyruvate de cyclohexylméthyle ou lesdits composés dans lesquels X = S.

21. Composition pharmaceutique selon l'une quelconque des revendications 17 à 19, dans laquelle, dans ladite substance, R3 ou R4 est OH, et est sélectionnée dans le groupe composé du D-énantiomère, du L-énantiomère et du mélange racémique de ceux-ci.

22. Composition pharmaceutique selon l'une quelconque des revendications 17 à 21, dans laquelle ladite substance selon la formule (I) est présente dans une concentration efficace sur le plan thérapeutique.

23. Composition pharmaceutique selon l'une quelconque des revendications 17 à 22, qui est destinée à une utilisation dans le traitement d'infections bactériennes associées à des biofilms.

24. Composition pharmaceutique selon la revendication 23, dans laquelle ledit biofilm est formé sur un support dans un environnement aquatique.

25. Composition pharmaceutique selon la revendication 24, dans laquelle ledit support est un ou plusieurs éléments sélectionnés parmi un matériel médical, des appareils ou des tissus médicaux.

26. Composition pharmaceutique l'une quelconque des revendications 24 à 25, dans laquelle ledit biofilm est associé à la formation de plaque dentaire.

27. Composition pharmaceutique l'une quelconque des revendications 17 à 26, qui comprend un ou plusieurs ingrédients supplémentaires actifs sur le plan pharmaceutique.

28. Composition pharmaceutique selon la revendication 27, dans laquelle ledit ingrédient supplémentaire actif sur le plan pharmaceutique comprend un composé qui stimule le métabolisme d'organismes infectieux.

29. Composition pharmaceutique selon la revendication 28, dans laquelle ledit composé qui stimule le métabolisme d'organismes infectieux est le glucose ou le 2,4-dinitrophénol.

30. Composition pharmaceutique selon la revendication 29, dans laquelle ledit ingrédient supplémentaire actif sur le plan pharmaceutique est sélectionné parmi des agents antibactériens.

31. Composition pharmaceutique selon la revendication 30, dans laquelle ledit agent antibactérien est un ou plusieurs éléments sélectionnés parmi les antibiotiques β-lactame, l'azithromycine, les aminoglycosides, les tétracyclines, un macrolide, les quinolones et les fluoroquinolones, les sulfonamides, les oxazolidones, les peptides biocides, le triméthoprime, le sulfaméthoxazole, le cloramphénicol, la vancomycine, le métronisazole et la rifampine.

32. Composition pharmaceutique selon l'une quelconque des revendications 17 à 31, qui comprend en outre une ou plusieurs substances auxiliaires, comprenant des charges, des agents d'aromatisation et des stabilisants.

33. Composition pharmaceutique selon l'une quelconque des revendications 17 à 32, dans laquelle ladite composition se présente sous la forme d'une formulation galénique à libération prolongée ou contrôlée.

34. Composition pharmaceutique selon l'une quelconque des revendications 17 à 33, qui est destinée à une administration topique ou systémique.

35. Composition pharmaceutique selon l'une quelconque des revendications 17 à 34, qui est destinée à une administration par voie orale, intraveineuse, sous-cutanée, intramusculaire, intradermique, intrapéritonéale, intra-auriculaire, rectale, intranasale, épidurale, percutanée, transdermique ou pulmonaire, ou à une administration sous la forme d'un aérosol, d'une huile, via des mini-pompes, en tant que rinçage buccal, crème, onguent, spray, gel, emplâtre et/ou via des microbulles.

36. Composition pharmaceutique selon l'une quelconque des revendications 17 à 35, qui est destinée à une utilisation en tant que complément alimentaire et/ou de complément pour boisson.

37. Composition pharmaceutique selon l'une quelconque des revendications 17 à 36, dans laquelle ledit animal est sélectionné parmi des invertébrés, des vertébrés non mammifères, et des mammifères, notamment des humains.

38. Composition pharmaceutique selon l'une quelconque des revendications 17 à 37, qui est destinée à une utilisation dans le traitement et/ou la prophylaxie d'infections bactériennes résistant à un traitement antibiotique.

39. Composition pharmaceutique selon la revendication 38, dans laquelle ladite résistance est une résistance à un ou plusieurs des éléments sélectionnés dans le groupe des pénicillines, des aminoglycosides, des tétracyclines, des macrolides, des fluoroquinolones, des sulfonamides, de la vancomycine, du triméthoprime, de la ciprofloxacine, des oxazolidinones, du linézolide, de la rifampine et des méthicillines.

40. Composition pharmaceutique selon l'une quelconque des revendications 17 à 39, qui est destinée à une utilisation dans le traitement et/ou la prophylaxie d'infections bactériennes par une bactérie aérobie et/ou anaérobie.

41. Composition pharmaceutique selon l'une quelconque des revendications 17 à 40, qui est destinée au traitement et/ou à la prophylaxie d'infections bactériennes par une bactérie Gram positive et/ou Gram négative.

42. Composition pharmaceutique selon l'une quelconque des revendications 17 à 41, dans laquelle ladite infection bactérienne est une ou plusieurs infections sélectionnées parmi une infection des voies respiratoires, comprenant les infections des voies respiratoires inférieures et supérieures, une infection cutanée, une infection intestinale, une infection gastrique, une infection systémique, comprenant des infections du tissu et du sang, est une parodontie ou une infection due à une implantation de dispositifs médicaux, notamment d'implants métalliques, d'articulations métalliques, ou est due à une cathétérisation.

43. Composition pharmaceutique selon l'une quelconque des revendications 17 à 42, qui est destinée à une utilisation dans le traitement d'infections par une ou plusieurs bactéries appartenant au genre Acrobacter, Actinobacillus, Actinomyces, Bacteroides, Borrelia, Bacillus, Brucella, Campylobacter, Clamydia, Clostridium, Corynebacterium, Cryptococcus, Enterobacter, Enterococcus, Erythrobacter, Eubacterium, Fusobacterium, cocci Gram positif, Heliobacter, Hemophilus, Lactobacillus, Legionella, Listeria, Mycobacteria, Mycoplasma, Neissaria, Pasteurella, Peptostreptococcus, Pneumococcus, Porphyromonas, Prevotella, Pseudomona, Pseudomonas, Rickettsia, Salmonella, Spirochetes Borrelia, Treponema, Staphylococcus, Streptococcus, Vibrio, Yersinia, et plus spécifiquement Escherichia coli, pneumocystis carinii, Helicobacter pylori ou Borrelia burgdorferi et/ou à une utilisation dans le traitement d'infections de bactéries appartenant à un ou plusieurs éléments du genre Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococcus aureus et Fusobacterium nucleatum.

44. Composition pharmaceutique selon l'une quelconque des revendications 17 à 43, dans laquelle ladite infection bactérienne est une infection opportuniste.

45. Composition pharmaceutique selon l'une quelconque des revendications 17 à 44, qui est destinée à une utilisation chez un animal immunosupprimé, notamment les humains.

46. Composition pharmaceutique selon la revendication 45, dans laquelle ladite immunosuppression est associée à des défauts immunitaires innés ou acquis.

47. Composition pharmaceutique selon la revendication 46, dans laquelle ledit défaut immun acquis est associé au VIH, à une transplantation d'organe, à une chimiothérapie ou à une exposition à un rayonnement.

48. Composition pharmaceutique selon l'une quelconque des revendications 17 à 47, qui est destinée à une utilisation chez un animal, notamment un humain, souffrant de façon concomitante d'une infection fongique ou à protozoaires.

49. Composition pharmaceutique selon l'une quelconque des revendications 17 à 48, qui est destinée à une utilisation chez un animal, notamment un humain, ayant un taux de glycémie sanguine réduit.

50. Composition pharmaceutique selon l'une quelconque des revendications 17 à 49, qui est destinée à une utilisation chez un animal, notamment un humain, souffrant de façon concomitante d'un cancer et/ou dans le cas où ledit animal, notamment un humain, va être soumis à, est en cours de, ou a été soumis à une thérapie anticancéreuse conventionnelle.

51. Composition pharmaceutique selon la revendication 50, dans laquelle ladite thérapie anticancéreuse conventionnelle est une ou plusieurs thérapies parmi une chimiothérapie, un traitement chirurgical, une radiothérapie ou une brachyradiothérapie.
